# EUROPÄISCHE PATENTSCHRIFT

(11) **EP 3 318 226 B1**
(45) Veröffentlichungstag und Bekanntmachung des Hinweises auf die Patenterteilung: **06.01.2021**
(21) Anmeldenummer: 16196998.5
(22) Anmeldetag: 03.11.2016
(51) Int. Cl.: A61F 9/007, A61M 1/00

(54) **SAUBERE VENTURI ASPIRATION**
CLEAN VENTURI ASPIRATION
ASPIRATION VENTURI PROPRE

(43) Veröffentlichungstag der Anmeldung: 09.05.2018
(73) Patentinhaber: This AG, 9435 Heerbrugg (CH)
(72) Erfinder: Köppel, Thomas, CH-9443 Widnau (CH)
(74) Vertreter: Kaminski Harmann

(56) Entgegenhaltungen:
- DE-A1-102007 042 964
- DE-T2- 69 032 869
- US-A- 5 746 719
- US-A1- 2013 023 821

## Beschreibung

Die Erfindung betrifft einen Wechsel-Einsatz bzw. eine Kassette für ein ophthalmologisches Medizinalgerät nach dem Oberbegriff des Anspruchs 1 sowie, ein entsprechendes Gerät oder System nach dem Oberbegriff des Anspruchs 11 und ein entsprechendes Aspirationsverfahren nach dem Oberbegriff des Anspruchs 13.

Bei Eingriffen im Auge, beispielsweise bei Katarakt-Vitrektomie- oder Glaucoma-Operationen, ist das chirurgische Instrument, mit welchem der Arzt den Eingriff im Auge selbst vornimmt über ein Schlauchsystem mit einem medizinischen Gerät verbunden, mit welchem - unter anderem - eine sogenannten Saugspülung durchgeführt wird, speziell um bei der Operation anfallende Partikel aus dem Auge zu entfernen. Dabei wird - meist mit einer oder mehreren, ins Auge eingeführten Hohlnadeln - eine Aspiration, also eine Absaugung, von den Partikeln und von Flüssigkeit vorgenommen. Zugleich wird mit einer Infusion Flüssigkeit, so genannte Irrigationsflüssigkeit, wieder zugeführt. Dabei soll der Augendruck möglichst konstant gehalten werden, um ein Einfallen oder Aufblähen des Auges zu verhindern. Die Dokumente WO 00/25710, WO 02/056805, DE 10 2012 018 982 oder DE 198 52 574 beschreiben Beispiele von derartigen Geräten und Beispiele von Anwendungen dieser Geräte.

Bei derartigen ophthalmologischen Saugspülungen sind primär zwei unterschiedliche Ansätze zur Aspiration in Verwendung. Zum einen, eine Absaugung mit einer Peristaltik-Pumpe, bei welcher - entsprechend deren Wirkungsweise - eine Vorgabe einer geförderten Volumenstrommenge bei deren Ansteuerung erfolgt. Zum anderen, eine Absaugung nach dem Venturi-Prinzip, bei deren Ansteuerung ein Unterdruck der Absaugung vorgegeben wird. Entsprechend sind entweder ophthalmologische Geräte nach dem Venturi-Prinzip, oder Geräte nach dem Peristaltik-Prinzip im Einsatz. Jedes dieser beiden Prinzipien hat seine spezifischen Charakteristiken bzw. Vor- und Nachteile, beispielsweise in Bezug auf Ripple-Effekte der Absaugung, Verhalten bei Okklusionen und deren Auflösungen, etc.. Welches dieser Prinzipien angewandt wird, hängt beispielsweise von den Vorlieben des Chirurgen und/oder der spezifischen Operation ab - oder schlichtweg von der Art des verfügbaren Geräts.

Beispielsweise zeigt WO 2010/129128 oder DE 10 2007 031 722 ein Gerät das nach dem Peristaltik-Prinzip arbeitet und US 2016/0089268 eine Anwendung des Venturi-Prinzips.

Beim Venturi-Prinzip, wird mittels einer Venturi-Düse (nach dem Venturi-Prinzip) mittels eines Luftstroms (beispielsweise von einem Druckluftanschluss im Operationssaal) in einer Kavität ein Unterdruck erzeugt. Diese Kavität wird bei Bedarf, beispielsweise über ein Ventil, mit dem vom Auge herführenden Aspirationssystem in Verbindung gebracht, und damit durch den Unterdruck die Aspiration bewirkt.

Jedoch weist ein ophthalmologisches Venturi-System nach dem Stand der Technik etliche Nachteile auf. Zum Beispiel kann der, für die Venturidüse erforderliche Schalldämpfer durch allfällige Keime aus der Augenflüssigkeit kontaminiert werden, und diese Keime, Vieren und/oder Bakterien können dann in die Umgebung ausgeblasen werden. Dies kann nicht nur auftreten wenn z.B. die Füllstandsbestimmung fehlschlägt und die Flüssigkeit überläuft, sondern kann auch schon durch kleinste Aufwirbelungen geschehen. Zwar sind spezielle, vorgeschaltete und regelmäßig zu wechselnde Filtersysteme bekannt, doch können diese nicht alle Infektionsgefahren verhindern. Auch die Tatsache, dass die aspirierte Flüssigkeit meist nur mit austauschbaren Kassetten in Berührung kommt, hilft hier wenig, da die Venturi-Düse, deren Schalldämpfer, etc. Teil des Geräts und nicht Teil der austauschbaren Kassette sind.

Ist bei einer derartigen Venturi-Kassette aus dem Stand der Technik, der zur Flüssigkeitsaufnahme vorgesehene Bereich voll, so muss zur Behebung dieses Problem die Operation unterbrochen werden, was einen weiteren Nachteil darstellt. Ein anderer Nachteil im Stand der Technik kann sein, dass bei Fehlfunktionen der Venturidüse ein enormer Überdruck im Aspirationssystem entstehen kann, welcher sich ins Auge übertragen und im schlimmsten Fall bis hin zu einem Einblasen von nichtsteriler Außenluft ins Auge führen könnte.

Da Venturi-Aspiration beliebt ist, beschreibt US 5,746,719 eine Einweg-Pump-Kassette für einen chirurgischen Eingriff, welche eine Rollen-Pumpe und einen Drucksensor aufweist. Um ein Venturi-ähnliches Aspirationsverhalten zumindest in etwa nachbilden zu können, wird vorgeschlagen, die Aspiration basierend auf Messwerten des Drucksensors, mit einem Ansteuerungs-Modul der Rollenpumpe und einer speziellen, inhaltlich nicht weiter erläuterten Funktion zu simulieren. Die bekannten Lösungen aus dem Stand der Technik sind daher in ihrer bekannten Form verbesserungswürdig.

Es ist eine Aufgabe der vorliegenden Erfindung, ein Verfahren bereitzustellen, welches eine ophthalmologische Aspiration verbessert. Es soll also ein verbessertes ophthalmologisches Aspirationsverfahren und die dabei verwendeten Vorrichtungen bereitgestellt werden. Insbesondere sollen oben genannte Nachteile ausgeräumt werden.

Es ist entsprechend auch eine Aufgabe der vorliegenden Erfindung, die ophthalmologische Aspirationseinrichtung zu verbessern, insbesondere den für die Aspiration vorgesehen Teilbereich eines ophthalmologischen Geräts und/oder den zugehörigen Wechselteil.

Dabei ist es insbesondere eine Aufgabe, eine entsprechende, verbesserte Kassette für ein ophthalmologisches Gerät bereitzustellen. Ebenfalls kann die entsprechende Bereitstellung eines ophthalmologischen Geräts zum Einsatz einer derartigen Kassette ein weiterer Teil der Aufgabe sein.

Eine spezifischere Aufgabe ist dabei auch, eine Lösung bereitzustellen, welche die von manchen Chirurgen geschätzten Eigenschaften und Charakteristiken eines bekannten Venturi-Systems aufweist, jedoch dessen Nachteile vermeidet oder zumindest reduziert.

Diese Aufgaben werden erfindungsgemäß durch die Merkmale der unabhängigen Ansprüche und/oder durch Merkmale der abhängigen Ansprüche gelöst bzw. diese Lösungen weitergebildet.

Die vorliegende Erfindung betrifft einen Wechsel-Einsatz für ein ophthalmologisches Medizinalgerät zur Aspiration von Flüssigkeit. Diese Aspiration kann insbesondere im Rahmen einer sogenannten Saugspülung bei einem ophthalmo-chirurgischen Eingriff, beispielsweise bei einer Katarakt- oder Vitrektomie-Operation, erfolgen. Bei dieser Aspiration ist Flüssigkeit - mit oder ohne Festkörperanteilen wie z.B. zertrümmerten Linsenteile oder ähnlichem - aus dem Auge abzuführen. Die Aspiration der vorliegende Erfindung soll dabei insbesondere primär nach dem in Fachkreisen so genannten "Venturi-Prinzip" erfolgen können, also nach dem Prinzip einer Absaugung mittels eines pneumatischen Unterdruckes und nicht nur nach einem hydraulischen Volumen-Förder-Prinzip, wie dies beispielsweise mit einer Peristaltikpumpe erzielt wird. Speziell also, um dabei eine, zu klassischen Venturi-Systemen ähnliche oder identische Absaugcharakteristik zu erzielen. Im Folgenden wird daher auch alternativ der Begriff "Clean-Venturi-Wechsel-Einsatz" für den Wechsel-Einsatz verwendet.

Der erfindungsgemäße Wechsel-Einsatz weist dabei einem Flüssigkeitsbereich zur Aufnahme der abzusaugenden Flüssigkeit sowie einen, insbesondere flüssigkeitsfreien, Unterdruckbereich, welcher mit einem pneumatischen Unterdruck beaufschlagbar ist, auf. Dabei ist der Flüssigkeitsbereich von dem Unterdruckbereich mit einer zumindest partiell flexiblen Membrane hermetisch getrennt.

Insbesondere bildet der Unterdruckbereich - speziell wenn das Wechselteil im Medizinalgerät eingelegt ist - einen Hohlraum aus. Dieser Hohlraum enthält dabei ein Gas, insbesondere Luft oder Raumluft. Aus diesem Hohlraum ist mittels einer entsprechenden Absaugeinrichtung das Gas (zumindest teilweise) absaugbar, sodass gegenüber der umgebenden Raumluft ein Unterdruck entsteht. Im regulären Betrieb enthält der Unterdruckbereich also üblicherweise keine Flüssigkeit.

Die Membrane kann dabei beispielsweise als dünne, flexible Folie ausgebildet sein, beispielsweise als Kunststofffolie oder als Metallfolie oder als eine Kombination dieser beiden. Die Membrane kann dabei beispielsweise aus einem Polyamid (PA) und/oder Polypropylen (PP), wobei je nach Material eine entsprechende Foliendicke durch Berechnung und/oder Versuche ermittelbar ist um die benötigte Flexibilität bei gleichzeitiger Stabilität zu erzielen. Beispielsweise ist eine Folie aus einem Polyamid (PA) und/oder Polypropylen (PP). Die Folie kann dabei mit einer Dicke von in etwa 40 bis 100 µm, speziell etwa mit 60 bis 70 µm ausgebildet sein. Neben einer Folie aus einem einzigen Material, kann die Folie auch aus zwei Materialien aufgebaut sein, z.B. etwa mit einer Schicht aus PA mit ca. 20 µm Dicke und einer Schicht aus PP mit 50 µm Dicke.

In einer Ausführungsform kann diese flexible Membrane sich beispielsweise zumindest annähernd vollflächig über eine Seitenwand des Flüssigkeitsbereichs erstrecken. Beispielsweise etwa über eine wannen- oder schalenförmig ausgestaltete harte Form, welche von der Membrane auf ihrer offenen Seite verschlossen wird. Dabei bilden die Form und die Membrane einen in seinem Volumen variablen Hohlraum, als zumindest einem Teil des Flüssigkeitsbereichs, aus. Das Volumen des Flüssigkeitsbereichs ist dabei durch zumindest partielle Verformung der Membrane veränderbar, insbesondere auch bis zu annähernd Null.

In einer anderen Ausführungsform kann sich diese flexible Membrane z.B. auch nur umlaufend um ein dadurch bewegliches festes, z.B. plattenförmiges Element, ausgebildet sein. Anders ausgedrückt, ist dabei im Allgemeinen die Membrane derart ausgebildet und angeordnet, um eine Beweglichkeit (zumindest eines Bereichs) einer oder mehrerer, der den Flüssigkeitsbereich umwandenden Seiten zu bewirken. Die flexible Membrane ist also speziell zur Veränderung des umschlossenen Volumens des Flüssigkeitsbereichs angeordnet und ausgebildet, speziell gegenüber einer nichtflexiblen Basis-Form des Wechselteils. Vorzugsweise ist dabei das ganze Wechselteil aus einem oder mehreren Kunststoffen gefertigt, z.B. um ein allfälliges Recycling zu vereinfachen.

Alternativ wären auch Ausführungsformen mit einer Kolben-Zylinder Anordnung realisierbar, bei welcher der Zylinder den Flüssigkeitsbereich bildet und der bewegliche Kolben anstelle der Membrane pneumatisch betätigt und zur Druckübertragung genutzt wird. Eine weitere Alternative, bei welcher ein Kolben oder eine steife Wand die via einer Membrane beweglich verbunden ist, mittels einer mechanisch oder magnetisch aufgebrachten Kraft bewegt wird. Letzteres würde (zumindest ohne spezielle Regelungstechnik) aber dem Prinzip der vorliegenden Erfindung widerstreben, nach welchem die Charakteristik eines Venturi-System möglichst beibehalten werden soll.

Erfindungsgemäß kann dabei beispielsweise der Wechsel-Einsatz mit einer Wanne oder Halbschale, also insbesondere einer Art schalenförmige Wanne mit einem Boden und einem umlaufend hochgestellten Rand, ausgebildet sein. Diese Halbschale kann beispielsweise aus einem Kunststoff - beispielsweise einem Polypropylen (PP) - oder einem Metall, und/oder einem thermoplastischen Elastomer (TPE) ausgebildet sein. Insbesondere bietet sich für eine Massenfertigung z.B. ein Spritzgussteil aus einem Polyethylen oder einem Polypropylen an. Beispielsweise kann die harte Komponente der Halbschale aus einem Polypropylen (PP) und die weiche Komponente aus einem TPE gebildet werden, vorzugsweise im Spritzgussverfahren. Mit dieser Halbschale kann dann - in hermetisch abschließender Weise - die Membrane verbunden sein. Die Membrane kann dabei zum Beispiel als dünne, flexible Folie ausgebildet sein, insbesondere mit einer derart geringen Foliendicke, dass die geforderte Flexibilität (zumindest partiell an den für die Volumenänderung erforderlichen Stellen oder auch über zumindest annähernd die gesamte Folienfläche) gegeben ist, zugleich aber eine hinreichende Reißfestigkeit sichergestellt ist. Die Folie kann dabei derart ausgebildet sein, dass diese sich in ihrer Ruhelage der inneren Form der Halbschale möglichst anschmiegt. Dies kann durch vorgängiges Formen oder beispielsweise auch bei der Herstellung des Wechsel-Einsatzes erfolgen. Beispielsweise kann die Halbschale mit der Folie überspannt und am Rand umlaufend versiegelt, verklebt, verschweißt, ultraschallgeschweißt, verpresst, vergossen oder geklemmt werden. Anschließend kann die Folie z.B. durch Tiefziehen in die Form der Halbschale in entsprechende Form gebracht werden - speziell derart, dass diese tiefgezogene Formgebung dann der bevorzugten Ruhelage der Membrane entspricht.

Eine derartige, flexible Folie kann dabei speziell derart angeordnet sein, dass zwischen einem Innenraum der Halbschale und einer Innenseite der flexiblen Folie der Flüssigkeitsbereich ausgebildet ist. Dieser Flüssigkeitsbereich hat dabei ein - je nach dessen Füllstand ein Volumen von zumindest annähernd Null bis hin zu etwa einigen zig bis einigen hundert Millilitern, beispielsweise von 100 ml. Speziell mit der zusätzlichen Pumpeinrichtung kann das Flüssigkeitsvolumen kleiner ausgebildet werden als ohne, beispielsweise mit weniger als 500 ml, vorzugsweise in etwa 100 ml.

Entsprechend wird von einer - der Innenseite gegenüberliegenden - Außenseite der Folie ein Teil des Unterdruckbereichs ausgebildet. Dabei kann zum Beispiel ein Rand der Halbschale bei eingelegter Kassette mit einem Gegenstück in dem Gerät eine umlaufend abgedichtete Kammer als Unterdruckbereich ausbilden, welche mit einer Luft-Absaugung zur Bereitstellung des Unterdrucks verbunden ist. Dabei kann beim Einlegen des Wechsel-Einsatzes insbesondere eine Klemmung zwischen dem Wechsel-Einsatz und dem Gegenstück erfolgen, mittels welcher ein geschlossenes Volumen gebildet wird. Dabei kann z.B. mit entsprechenden Dichtelementen an dem Wechselteil und/oder an dem Gegenstück im Gerät, der Unterdruckbereich als (bis auf die Absaugung) geschlossenes Volumen gebildet werden, insbesondere ein hermetisch geschlossenes Volumen. Eine Ausführungsform des erfindungsgemäßen Wechselteils kann dabei beispielsweise als Mehrkomponenten-Spritzgussteil ausgebildet sein, bei welchem die Folie eingelegt oder optional mitgespritzt wird. Entsprechend ist dabei auf die bekannte Kombinierbarkeit der verwendeten Materialien in diesem Verfahren zu achten.

Der erfindungsgemäße Wechsel-Einsatz kann in einer Ausführungsform beispielsweise als austauschbare Kassette für das ophthalmologisches Medizinalgerät ausgebildet sein. Der Flüssigkeitsbereich ist dabei als eine mit einem Aspirationsanschluss eines chirurgischen Operations-Bestecks hydrostatisch verbindbare Saugkammer ausgebildet. Diese Saugkammer ist dabei zur zumindest temporären, insbesondere direkten, Aufnahme von aspirierter Flüssigkeit ausgebildet und wird - wie bereits beschrieben - im Wesentlichen mit der flexiblen Membrane und der festen Halbschale ausgebildet. Dabei ist die Membrane - wenn die Kassette im Medizinalgerät eingelegt ist - von außen über den Unterdruckbereich mit einem Unterdruck beaufschlagbar, sodass eine Übertragung des Unterdrucks vom Unterdruckbereich via der Membrane auf den Flüssigkeitsbereich erfolgen kann, ohne dass die aus dem Unterdruckbereich abgesaugte Luft in Berührung mit der Flüssigkeit kommen kann. Dabei ist insbesondere bei entsprechender Auslegung des Materials und der Parameter, insbesondere der Folienstärke, der Membrane eine Aspirationscharakteristik, welche gleich oder zumindest ähnlich jener von bekannten Venturi-Aspirationssystemen ist. Insbesondere kann dabei die Membrane eine großflächige Membrane sein, was speziell bedeuten kann, dass die Fläche der Membrane einen wesentlichen Anteil an der, den Flüssigkeitsbereichs umschließenden Oberfläche ausmacht. Beispielsweise kann die Fläche der Membrane im Verhältnis zur gesamten Oberfläche des Flüssigkeitsbereiches dabei nicht nur lediglich im einstelligen Prozentbereich liegen, sondern z.B. mehr als 16%, insbesondere mehr als 25% oder mehr als 35% oder noch mehr ausmachen. Mit einer entsprechend dimensionierten, großflächigen Membrane kann dabei insbesondere eine, einem bekannten Venturi-System besonders ähnliche Charakteristik erzielt werden. Optional kann in einer speziellen Ausführungsform die Membranfläche auch bis hin zu annähernd 100% ausmachen, wobei jedoch vorzugsweise ein Prozentsatz von unter 80% angewandt wird, sodass zumindest ein Teil der Oberfläche des Flüssigkeitsbereichs von der festen Halbschale gebildet wird.

Der erfindungsgemäße Wechsel-Einsatz kann in einer anderen Ausführungsform auch als ein optionales Zusatzmodul ausgebildet sein, welches an eine ophthalmologische Basis-Kassette - welche beispielsweise bereits zumindest eine Peristaltikpumpe, Anschlüsse oder Ventile aufweist - anbringbar ist. Beispielsweise kann dabei das optionale Zusatzmodul und die Basis-Kassette miteinander fest verbindbar sein (z.B. verschnappbar, klemmbar, verriegelbar, etc.), sodass bei der Anbringung eine hydraulische Verbindung des Flüssigkeitsbereichs mit einem Aspirationssystem der Basis-Kassette herstellbar ist. Das Anbringen kann vor dem Einlegen der Kassette - also beispielsweise im OP - erfolgen, indem die Basis-Kassette sowie bei Bedarf der Clean-Venturi-Wechsel-Einsatz aus ihrer sterilen Verpackung entnommen und vor Ort, vorzugsweise unlösbar, zusammengefügt werden. Erfolgt die Anbringung des Zusatzmoduls bereits ab Werk, kann alternativ auch eine Verklebung, Verpressung, Verschweißung oder eine formschlüssige Verbindung mit der Basis-Kassette erfolgen, und die entstandene, unlösbar verbundene Wechsel-Kassette mit erfindungsgemäßem Clean-Venturi-Wechsel-Einsatz gesamt steril verpackt werden, z.B. in einem Blister. Beim Zusammenfügen kann dabei beispielsweise ein Ausbrechteil oder Siegel der Basis-Kassette verletzt werden um eine Verbindung zu deren Aspirationssystem bereitzustellen, oder die Basis-Kassette kann ein Ventil oder dergleichen zur Freigabe einer Verbindung eines Flüssigkeitskanals hin zum Clean-Venturi-Wechsel-Einsatz aufweisen.

Beim erfindungsgemäßen Wechsel-Einsatz kann der Unterdruck von einer Luft-Absaug-Einrichtung im Medizinalgerät erzeugt werden. Dabei kann beispielsweise eine elektrisch betriebenen Teil-Vakuum-Pumpe angewandt werden, oder allenfalls auch eine Venturi-Düse oder eine andere Luft-Fördereinrichtung, mit welcher ein Unterdruck erzeugbar ist. (Teil-Vakuum, da erfindungsgemäß meist kein vollständiges Vakuum, sondern nur ein hinreichender Unterdruck gegenüber dem Atmosphärendruck erforderlich ist). Eine Vakuumpumpe bietet meist den Vorteil eines leisen und wartungsarmen Betriebs und vermeidet Probleme eines unerwünschten Überdrucks im Falle einer Verstopfung. Speziell kann die Luft-Absaug-Einrichtung dabei derart ausgebildet sein, dass der erzeugte Unterdruck in seinem Wert einstellbar oder regelbar ist. Typische Wertebereiche für den Unterdruck im Sinne der vorliegenden Erfindung sind beispielsweise in etwa von 0 bis 850 mbar unter Atmosphärendruck. Falls im Operationssaal ein Vakuumanschluss vorhanden ist, kann optional anstelle einer geräteseitigen Luft-Absaug-Einrichtung auch eine Verbindung mit diesem herstellbar sein oder es kann eine Druck-Vakuum Umwandlung mittels Venturi-Düse erfolgen.

Im Gerät ist dabei ein Teil der Vakuumeinrichtung, bzw. ein mit dieser funktional in Verbindung stehender Teil, als Gegenstück ausgebildet, welches umlaufend mit einem Rand des Wechselteils, speziell der Halbschale und/oder der Membrane bei eingelegtem Wechselteil dichtend abschließt. Dabei wird zwischen Gerät und dem Wechselteil der Unterdruckbereich ausgebildet. Insbesondere kann dabei am Wechselteil, speziell an dessen Rand, und/oder am Gegenstück im Gerät eine Dichtung vorhanden sein, beispielsweise mit einem Elastomer, einem thermoplastischen Elastomer, einem Silikon oder einer anderen bekannte Dichtungsform. Speziell kann dabei beim oder nach dem Einlegen des Wechselteils in das Gerät eine Klemmkraft zwischen dem Gegenstück und dem eingelegten Wechselteil aufgebracht werden, mittels welcher die Dichtung ihre Wirkung entfaltet.

Bei dem erfindungsgemäßen Wechsel-Einsatz kann dabei der Flüssigkeitsbereich eine Druckerfassungseinrichtung aufweisen oder mit einer derartigen in hydrostatischer Verbindung stehen. Diese ist derart ausgebildet, um einen Unterdruck und/oder Überdruck (absolut oder vorzugsweise relativ gegenüber der umgebenden Atmosphäre) in dem Flüssigkeitsbereich zu erfassen. Dabei kann die Druckerfassungseinrichtung neben einer kompletten Integration in den Wechsel-Einsatz auch vorzugsweise nur teilweise an diesem angebracht sein. So kann beispielsweise das Wechselteil nur eine, mit dem Flüssigkeitsbereich in Verbindung stehende Druckmessmembrane aufweisen, und ein zugehöriges Messglied, welches eine Kraft oder eine Auslenkung der Druckmessmembrane bestimmt, im Gerät angebracht sein. Dieses Messglied kann beim Einlegen des Wechselteils mit der Druckmessmembrane eine funktionale, insbesondere mechanische, Verbindung eingehen. In analoger Weise kann auch zusätzlich oder alternativ eine weitere Druckerfassungseinrichtung für den Unterdruckbereich vorgesehen sein, welche entsprechend dem Gerät oder dem Wechselteil zugeordnet sein kann. Eine derartige Druckerfassungseinrichtung kann dabei unter anderem für verschiedenste Überwachungs- und Sicherheits- oder Regelungs-Funktionen genutzt werden.

Bei dem erfindungsgemäßen Wechsel-Einsatz bzw. bei der Kassette kann in einem Flüssigkeitspfad zwischen dem Flüssigkeitsbereich und einem Anschluss für eine Aspirationsleitung zumindest ein Ventil vorgesehen sein. Dieses kann insbesondere derart ausgebildet sein, dass damit ein Querschnitt des Flüssigkeitspfades veränderbar ist, also beispielsweise freigegeben oder verschlossen werden kann, optional aber auch Zwischenstellungen einnehmen kann. Beispielsweise kann ein derartiges Ventil als ein Querschnittsbereich des Flüssigkeitspfades ausgestaltet sein, welcher Querschnittsbereich entlang seines Umfangs zumindest teilweise elastisch ausgebildet ist. Dieser elastische Bereich kann dann durch einen Stößel, einen Schieber oder dergleichen derart betätigt werden, dass dadurch der freie Querschnittsbereich im Flüssigkeitspfad veränderbar ist, insbesondere geöffnet oder verschlossen werden kann.

Der erfindungsgemäße Wechsel-Einsatz bzw. die Kassette kann auch eine Pumpvorrichtung aufweisen, insbesondere eine Peristaltikpumpe oder Membranpumpe. Mit dieser Pumpvorrichtung kann beispielsweise Flüssigkeit aus dem Flüssigkeitsbereich in einen austauschbaren Abfallbehälter oder Auffangbeutel abführbar sein. Dieses Abführen kann beispielsweise erfolgen, wenn der Flüssigkeitsbereich bis zu einer bestimmten Maximalkapazität gefüllt ist, oder kann optional aber auch fortlaufend während der Aspiration oder während Aspirationspausen erfolgen, vorzugsweise automatisch. Dazu kann der Aspirationsanschluss in seiner Flüssigkeitsverbindung von dem Flüssigkeitsbereich abtrennbar sein, beispielsweise mittels eines dafür vorgesehenen Ventils, um bei der Abführung Druckänderungen des Aspirationssystems beim Patienten zu vermeiden. Diese Pumpvorrichtung kann dabei optional auch in ihrer Flüssigkeitsverbindung von dem Flüssigkeitsbereich abtrennbar sein, beispielsweise mittels eines dafür vorgesehenen weiteren Ventils. Optional kann aber im Allgemeinen beispielsweise auch eine stehende, sich im Eingriff befindliche Peristaltikpumpe als ein geschlossenes Ventil wirken, insbesondere wenn zumindest eine Pumpen-Rolle den Pumpquerschnitt vollständig quetscht. Der Abfallbehälter kann z.B. ein austauschbarer Beutel oder Kanister sein, welcher vorzugsweise extern vom Gerät und/oder der Kassette angebracht werden kann. Dies kann z.B. über eine dafür vorgesehene Anschlussleitung oder vorzugsweise ohne Schlauch mit einem direkt an der Kassette anbringbaren Beutel erfolgen.

Im erfindungsgemäßen Wechsel-Einsatz können Flüssigkeitspfade derart ausgebildet sein, dass, insbesondere mittels zumindest eines dafür vorgesehenen Ventils, eine Umschaltung zwischen einer Aspiration mittels des auf den Flüssigkeitsbereich übertragenen Unterdrucks, oder einer alternativen Pumpvorrichtung, insbesondere einer Peristaltikpumpe, oder auch einer Kombination dieser beiden durchführbar ist. Dabei kann als die alternative Pumpvorrichtung insbesondere dieselbe Pumpvorrichtung genutzt werden, mit welcher wie zuvor beschrieben auch Flüssigkeit in den Abfallbehälter abführbar ist. Optional kann eine derartige alternative Pumpvorrichtung auch als Rückfalloption genutzt werden, beispielsweise falls der Flüssigkeitsbereich voll ist - aber eine Weiterführung der Aspiration erforderlich ist. Auch bei Ausfall eines der beiden Aspirationssysteme kann das andere vorzugsweise nahtlos übernehmen. Eine weitere Option ist, dass mittels eines Zusammenwirkens von der erfindungsgemäßen Clean-Venturi-Aspiration und der Pumpvorrichtung ein Fördervolumen- und/oder Aspirationsdruck-Boost erzielt wird, was in gewissen Situationen während einer Operation hilfreich sein kann. Auch kann der Bediener in einer entsprechenden Ausführungsform damit verzögerungsfrei und vorzugsweise stufenlos zwischen einer Peristaltik- oder Venturi-Aspiration oder einer beliebigen Kombination dieser beiden Modi umschalten. Auch kann mit entsprechender Ansteuerung eine Rückfluss-Funktion realisiert werden, was sich mit einer reinen Venturi-Lösung aufwändiger gestalten würde. Neben dem Wert des Unterdruckes kann auch mittels Auslegung und/oder Variation des Volumens des Unterdruckbereichs eine Anpassung der Absaugcharakteristik erzielt werden, beispielsweise etwa das Verhalten bei Okklusionen, etc. beeinflusst werden. Beispielsweise bewirkt ein größeres Volumen des Unterdruckbereichs eine größere Elastizität des Saugverhaltens. Bei einer erfindungsgemäßen Ausführungsform, insbesondere bei einer Ausführungsform mit Clean-Veturi als auch Pumpvorrichtungs-Aspiration, kann somit eine hohe, und insbesondere während des Betriebs einstellbare oder umstellbare, Dynamik der Aspiration erzielt werden. Eine Variation des Dynamik-Verhaltens der Aspiration kann erfindungsgemäß beispielsweise auch durch Beeinflussung des Unterdrucks, insbesondere in seinem zeitlichen Aufbau, erzielt werden.

Der erfindungsgemäße Wechsel-Einsatz und/oder das Medizingerät mit welchem dieser zusammenwirkt, kann auch zumindest einen Füllstands-Sensor für den Flüssigkeitsbereich aufweisen. Dieser kann insbesondere als ein Drucksensor ausgebildet sein, oder als ein Sensor zur Bestimmung einer Position der Membrane. Beispielsweise kann der Füllstands-Sensor als ein bei gefülltem Flüssigkeitsbereich durch die Membrane betätigter Schalter ausgebildet sein. Bei vollem Flüssigkeitsbereich kann dann entweder eine Aufforderung zum Wechsel des Wechsel-Einsatzes erfolgen, oder ein oben beschriebenes Abführen von Flüssigkeit aus dem Flüssigkeitsbereich mittels einer Pumpvorrichtung erfolgen. Eine Detektierung eines vollständig entleerten Flüssigkeitsbereichs des Wechsel-Einsatzes kann entweder durch einen zusätzlichen oder denselben Füllstands-Sensor erfolgen, oder dies kann z.B. mittels der weiter oben bereits erwähnten Druckerfassungseinrichtung detektiert werden.

Auch kann ein erfindungsgemäßes Wechselteil oder eine erfindungsgemäße Kassette einen Luftblasen-Detektor aufweisen. Dieser kann unter anderem beispielsweise auf optischer Basis arbeiten, speziell auf Basis von unterschiedlicher Refraktion von Flüssigkeit und Luft. Beispielsweise kann an der Kassette oder am Wechselteil ein Sichtfenster in den Flüssigkeitskanal implementiert sein, durch welches ein geräteseitiger optischer Sensor allenfalls auftretende Luftblasen detektiert.

Ebenso betrifft die vorliegende Erfindung ein entsprechendes Gerät oder System zur Augenoperation mit einer Aufnahmevorrichtung für zumindest eine der Ausführungsformen eines erfindungsgemäßen Wechsel-Einsatz oder einer erfindungsgemäßen Kassette, speziell wie diese hier beschrieben sind.

Das Gerät weist dabei eine Luft-Absaug-Einrichtung, insbesondere eine Vakuumpumpe bzw. eine Teil- oder Grob-Vakuum-pumpe oder eine Venturi-Düse, zur Erzeugung eines Unterdrucks im Unterdruckbereich auf. Insbesondere also eine Einrichtung zur Abpumpung oder Absaugung von Luft aus dem, vom Flüssigkeitsbereich des Wechsel-Einsatzes hermetisch getrennten, Unterdruckbereich des Wechsel-Einsatzes. Dabei kann die Aufnahmevorrichtung für den Wechsel-Einsatz insbesondere derart ausgebildet sein, dass - bei aufgenommenem Wechsel-Einsatz - ein hermetisch abschließendes, lösbares Fügen eines umlaufenden Bereichs des Wechsel-Einsatz mit einem entsprechenden Gegenstück der Aufnahmevorrichtung des Geräts zur Bildung des Unterdruckbereichs erfolgt. Der mit Wechsel-Einsatz und Gerät gebildete Unterdruckbereich kann dabei insbesondere einen - allenfalls abgesehen von der Luft-Absaug-Einrichtung selbst - geschlossenen Volumenbereich darstellen, welcher eine äußere, insbesondere von Außen zugängliche, Seite der Membrane des Wechselteils mitumfasst. Beispielsweise kann dabei eine umlaufende Linie oder Fläche als Dichtbereich ausgebildet sein, insbesondere mit einer Dichtung, vorzugsweise mit einem Elastomer oder thermoplastischen Elastomer, am Wechselteil und/oder dem entsprechenden Gegenstück im Gerät. Der Unterdruckbereich kann beispielsweise zudem auch einen Lecksensor, beispielsweise mittels eines Flüssigkeits- oder Feuchtigkeitssensor und/oder einen Drucksensor aufweisen, mit welchen eine Fehlfunktion oder ein Defekt einer Abdichtung, der Membrane, etc. mittelbar oder unmittelbar detektierbar ist. Der Anschluss der Absaug-Einrichtung kann dabei insbesondere im oberen Bereich des Unterdruckbereichs erfolgen, sodass im Falle einer Leckage keine Flüssigkeit in die Absaug-Einrichtung eindringen kann. Die hydraulische Verbindung von Flüssigkeitsbereich zum Aspirationssystem kann dabei vorzugsweise in einem oberen Bereich des eingelegten Wechselteils erfolgen, speziell um damit allfälliger Luftblasenbildung vorzubeugen, beziehungsweise diese Luftblasen einfach abführen zu können. Vorzugsweise ist die Membrane dabei derart ausgebildet, dass diese in ihrer Ruhelage, speziell nach dem Auspacken des Wechselteils, das Volumen des Flüssigkeitsbereichs bei annähernd Null hält. Insbesondere kann nach dem ersten Einlegen - also bei der Inbetriebnahme des Wechsel-Teils - ein so genannter Füll- oder Spül-Zyklus gefahren werden, bei welchem erst Aspirations- oder vorzugsweise Infusionsflüssigkeit in den Flüssigkeitsbereich eingesaugt wird und anschließend ein Abführen dieser Flüssigkeit gemeinsam mit allfällig im Aspirationssystem befindlicher Luft aus dem Wechsel-Einsatz, respektive der Kassette und/oder des Schlauchsystems erfolgt. Beispielsweise kann das Abführen in einen Auffangbeutel erfolgen. Dies ist insbesondere vorteilhaft, da ein gänzlich mit Flüssigkeit gefülltes Aspirationssystem deterministischer auf Druckänderungen reagieren kann.

In analoger Weise betrifft die Erfindung auch ein Aspirationsverfahren für ein ophthalmologisches Gerät, insbesondere ein Aspirationsverfahren nach einem auf Basis von Unterdruck arbeitenden Venturi-Prinzip. Dieses Verfahren kann insbesondere im Rahmen einer Saugspülung bei einer Katarakt-Operation, beispielsweise bei einer Phakoemulsifikation oder Vitrektomie durchgeführt werden. Das erfindungsgemäße Verfahren erfolgt dabei mit einem Bereitstellen eines hermetisch abgeschlossenen Flüssigkeitsbereichs, welcher zumindest teilweise von einer flexiblen Membrane gebildet wird, und welcher Flüssigkeitsbereich sich in einem Wechsel-Einsatz für das ophthalmologisches Gerät befindet und mit einem Aspirationsanschluss hin zum Patienten in hydraulische Verbindung bringbar ist.

Das Aspirieren erfolgt dabei zumindest teilweise mit einem Absaugen oder Abpumpen von Luft aus einem, gegenüber dem Flüssigkeitsbereich auf einer Außenseite der Membrane ausgebildeten, Unterdruckbereich. Dabei erfolgt mittels der Membrane eine Übertragung des pneumatischen Unterdrucks im Unterdruckbereich auf einen hydraulischen Unterdruck im Flüssigkeitsbereich. Das bilden des Unterdruckbereichs kann dabei durch ein hermetisch abdichtendes Fügen des Wechselteils an ein geräteseitiges Gegenstück erfolgen, wobei das Gegenstück mit einer Einrichtung zur Erzeugung des Unterdruckes ausgebildet oder verbunden ist.

In anderen Worten handelt es sich um ein Aspirationsverfahren für eine ophthalmologische Operation nach dem Venturi Prinzip, insbesondere bei einem Phakogerät. Bei diesem erfolgt erfindungsgemäß eine hermetische Trennung eines Flüssigkeitsbereichs für die aspirierte Flüssigkeit von einem mit der Raumluft in Verbindung stehenden Unterdruckbereich. Es erfolgt also erfindungsgemäß ein Übertragen eines pneumatischen Unterdrucks zu einem hydraulischen Unterdruck des Aspirationssystems durch eine flexible Membrane. Dabei kann insbesondere der Unterdruckbereich mit einem definierten Mindestvolumen ausgelegt sein, und damit durch dessen pneumatische Wirkung eine dynamische Charakteristik der Aspiration bewirkt wird, welche vorteilhaft gegenüber einer starren Peristaltik-Aspiration sein kann.

Das erfindungsgemäße Aspirationsverfahren kann mit einem Messen eines Druckes im Flüssigkeitsbereich und/oder im Unterdruckbereich erfolgen, insbesondere wobei damit eine Regelung des Unterdruckes und/oder der Ventilansteuerung erfolgt. Das erfindungsgemäße Aspirationsverfahren kann auch mit einem Umschalten zwischen einer Aspiration mittels des pneumatischen Unterdrucks, welcher auf den Flüssigkeitsbereich wirkt, und/oder mittels einer hydraulischen Volumenförderung, insbesondere durch ein peristaltisches Pumpen, erfolgen. Beim erfindungsgemäßen Aspirationsverfahren kann zudem ein Abführen von Flüssigkeit aus dem Flüssigkeitsbereich in einen Abfallbehälter, insbesondere durch ein peristaltisches Pumpen, erfolgen. Dabei kann insbesondere ein, vorzugsweise fortlaufendes, Entleeren des Flüssigkeitsbereichs während Aspirationspausen erfolgen.

Das erfindungsgemäße Verfahren und die erfindungsgemäße Vorrichtung werden nachfolgend anhand von in den Zeichnungen schematisch dargestellten, konkreten Ausführungsbeispielen rein beispielhaft näher beschrieben, wobei auch auf weitere Vorteile der Erfindung eingegangen wird. Im Einzelnen zeigen:
Fig. 1 eine erste Ausführungsform eines Beispiels eines erfindungsgemäßen Wechsel-Einsatz als Block-Schema;
Fig. 2 eine zweite Ausführungsform eines Beispiels eines erfindungsgemäßen Wechsel-Einsatz als Block-Schema;
Fig. 3 eine Schnitt-Darstellung einer beispielhaften ersten erfindungsgemäßen Ausführungsform eines Wechsel-Einsatzes;
Fig. 4 eine Explosions-Darstellung einer beispielhaften zweiten erfindungsgemäßen Ausführungsform eines Wechsel-Einsatzes;
Fig. 5a eine erste Darstellung einer beispielhaften dritten erfindungsgemäßen Ausführungsform eines Wechsel-Einsatzes in Mehrkomponenten-Spritzguss Ausführung;
Fig. 5b eine zweite Darstellung der beispielhaften Ausführungsform eines erfindungsgemäßem Wechsel-Einsatzes in Mehrkomponenten-Spritzguss Ausführung;
Fig. 6a eine erste Darstellung einer ersten beispielhaften Ausführungsform einer erfindungsgemäßen ophthalmologischen Kassette mit erfindungsgemäßem Wechsel-Einsatz;
Fig. 6b eine zweite Darstellung der ersten beispielhaften Ausführungsform einer erfindungsgemäßen ophthalmologischen Kassette mit erfindungsgemäßem Wechsel-Einsatz;
Fig. 7 eine perspektivische Darstellung einer beispielhaften Ausführungsform einer Venturi-Membrane eines erfindungsgemäßen Wechsel-Einsatzes;
Fig. 8a eine Schnitt-Darstellung einer beispielhaften Ausführungsform einer Venturi-Wanne eines erfindungsgemäßen Wechsel-Einsatzes;
Fig. 8b und Fig. 8c eine Detail-Darstellung einer beispielhaften Ausführungsform einer Venturi-Wanne als Halbschale eines erfindungsgemäßen Wechsel-Einsatzes;
Fig. 9 eine Darstellung einer beispielhaften Ausführungsform eines erfindungsgemäßen Abfallbeutels für einen erfindungsgemäßen Wechsel-Einsatz;
Fig. 10 eine Darstellung eines Ausschnitts eines erfindungsgemäßen Geräts mit eingelegter erfindungsgemäßer Kassette und angebrachtem erfindungsgemäßen Abfallbeutel.

Die Darstellungen in den Figuren dienen lediglich zur Illustration und sind, sofern nicht explizit angegeben, nicht als maßstäblich zu betrachten. Gleiche oder funktionell ähnliche Merkmale sind, insoweit praktikabel, durchgängig mit denselben Bezugszeichen versehen, und werden gegebenenfalls mit einem Buchstaben als Index unterschieden. Die dargestellten Schemata zeigen jeweils die technische Grundstruktur, welche von einem Fachmann entsprechend allgemeinen Grundsätzen ergänzt oder modifiziert werden können.

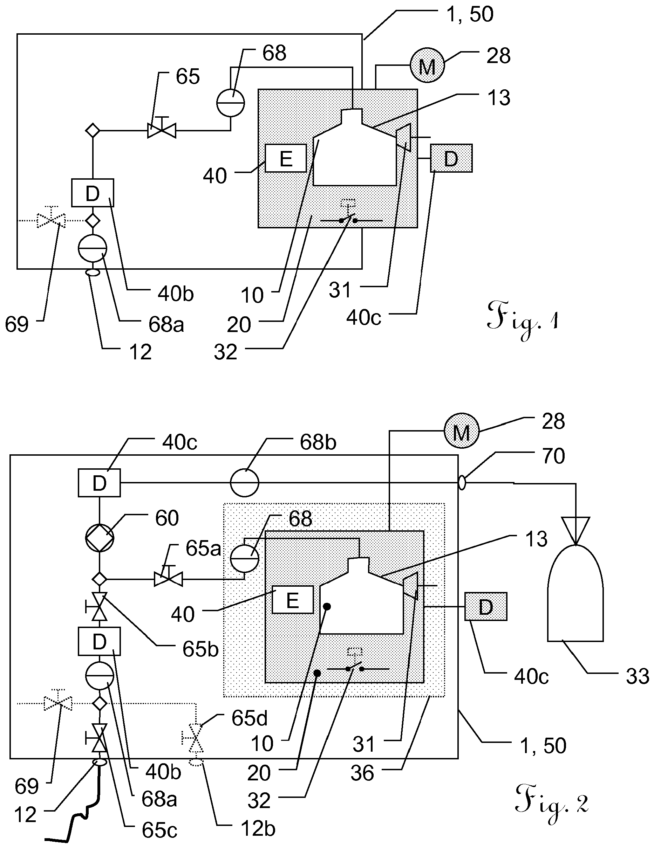

**Fig. 1** zeigt eine Ausführungsform eines erfindungsgemäßen Aspirationsverfahrens bzw. eines entsprechenden Wechsel-Einsatzes 1 als vereinfachtes Blockdiagramm. Die folgende Beschreibung folgt dabei dem Pfad der aspirierten Flüssigkeit, wobei - insbesondere optionale - Elemente im Sinne der Erfindung auch in ihrer Reihenfolge variierbar sind.

Der Aspirationsanschluss 12 ist zur Verbindung mit einem ophthalmologisch-chirurgischen Instrument, beispielsweise einem Phako-Handstück, von dem aspiriert werden soll, als Schnittstelle des Wechsel-Einsatzes 1, welcher auch als austauschbare Kassette 50 für ein ophthalmologisches Gerät ausgebildet sein kann. Im hier gezeigten Beispiel befindet sich in der kassetteninternen Aspirationsleitung ein optionaler Luft-Detektor 68a, mittels welchem allfällige Luftblasen im Aspirationssystem feststellbar sind. Mit dem optionalen Ventil 69 kann in dieser beispielhaften Ausführungsform Infusionsflüssigkeit in das Aspirationssystem geleitet werden, beispielsweise zur Befüllung der Kassette 50 mit Flüssigkeit bei deren Inbetriebnahme und/oder zum Unterdruckabbau bei Okklusionen im Aspirationssystem. Weiters weist das Aspirationssystem einen Drucksensor 40b auf, mit welchem der Aspirationsdruck, insbesondere als Unter- und Überdruck gegenüber der Atmosphäre, überwacht werden kann. Für die Anwendung erforderlich ist das Ventil 65, mittels welchem die Aspiration ein- und ausgeschaltet, bzw. geregelt werden kann. Der hier gezeigte, optionale Drucksensor 40 kann zur Messung, Überwachung und/oder Regelung des Aspirationsunterdruckes, und/oder zur Füllstandsüberwachung des Flüssigkeitsbereichs 10 genutzt werden. Bei gefülltem Flüssigkeitsbereich 10 oder bei einer Fehlfunktion lässt sich beispielsweise trotz Unterdruck im Unterdruckbereich 20 kein Unterdruck im Aspirationssystem bewirken. Ebenfalls optional kann nochmals ein Luft-Detektor 68 vorhanden sein. Im Anschluss gelangt die aspirierte Flüssigkeit in den Flüssigkeitsbereich 10 des Wechselteils, welcher eine flexible Membrane 13 aufweist. Diese flexible Membrane 13 befindet sich zumindest teilweise in einem Unterdruckbereich 20, welcher hermetisch vom Flüssigkeitsbereich getrennt ist. Für den Fehlerfall einer Leckage des Flüssigkeitsbereichs 10 kann der Unterdruckbereich 20 mit einem optionalen Feuchtigkeits-Detektor 32 ausgestattet sein. Auch kann der Unterdruckbereich 20 oder der Flüssigkeitsbereich 10 mit einem optionalen Füllstandssensor 31 für den Flüssigkeitsbereich 10 ausgestattet sein. Der Unterdruckbereich 20 wird dabei von einem Unterduck beaufschlagt, welcher durch Abführen der im Unterdruckbereich 20 vorhandenen Luft erzeugt wird, beispielsweise durch eine Gas-Fördereinrichtung 28 wie der gezeigten (Teil-) Vakuum-pumpe 28, oder einer anderen, gleichwirkenden Einrichtung. Dabei kann optional ein Drucksensor 40c zur Messung, Überwachung und/oder Regelung des von der Gas-Fördereinrichtung 28 erzeugten Unterdruckes vorhanden sein, insbesondere kann der Unterdruck also einstell- und/oder regelbar sein.

Beim Aufbau können auch Teile der zur Erzielung oben genanter Funktionalitäten benötigte Elemente zumindest Teilweise dem Gerät, in welchem das Wechselteil 1 für dessen Betrieb eingelegt wird, angeordnet sein. Insbesondere können dies die aktiven Teile der Aktoren oder Sensoren sein, welche mit der Kassette zusammenwirken - Jedoch sind, dem Zweck des Wechselteils folgend, sämtliche im regulären Betrieb mit der Flüssigkeit in Verbindung kommende Bereiche in der Kassette (bzw. Außerhalb des Geräts) anzuordnen. Die Kassette enthält somit vorzugsweise möglichst nur die passiven Teile dieser Elemente. Erfindungsgemäß können dabei insbesondere Betätigungselemente für die Ventile 65, Messelektronik für die Sensoren 40, 40b, 40c, 68, 31, 32, die Gas-Fördereinrichtung 28, etc. im Gerät, in welches das Wechselteil eingelegt wird, angeordnet sein. Insbesondere kann auch zumindest ein Teil des Unterdruckbereichs 20 von einem geräteseitigen Gegenstück zur Kassette 50 gebildet werden. Beispielhaft sind vorzugsweise im Gerät befindliche Blöcke im Diagramm hinterlegt gezeichnet, wobei dies nur einer möglichen Ausführungsform entspricht und nicht zwingend als einschränkend gesehen werden muss.

Aus Sicht einiger der weiter oben genannten Vorteile gegenüber der bekannten ophthalmologischen Venturi-Aspirations-Lösungen aus dem Stand der Technik, kann die vorliegende Erfindung auch als "Clean-Venturi" bezeichnet werden, da diese das Verhalten eines klassischen Venturi Systems ohne die oben genannten Nachteile bereitstellt, insbesondere ohne die Verschmutzungsproblematik.

**Fig. 2** zeigt eine Ausführungsform einer erfindungsgemäßen Clean-Venturi-Aspiration bzw. einer entsprechenden ophthalmologischen Kassette 50 als vereinfachtes Blockdiagramm. Die Figur stellt primär eine Erweiterung der oben genannten Funktionalität dar, sodass auf gleichbleibende Elemente nicht nochmals spezifisch eingegangen, sondern auf oben verwiesen wird. Im Speziellen kommt in dieser erweiterten erfindungsgemäßen Ausführungsform der ophthalmologischen Kassette 50, eine zusätzliche Pumpeinrichtung 60, beispielsweise eine Venturi oder Membranpumpe, im Aspirationssystem hinzu. Weiters ist in der gezeigten Ausführungsform ein zusätzliches Ventil 65b und ein optionaler Luftblasen-Detektor 68b gezeigt. Mittels diesen Elementen kann zusätzlich zu bei Fig. 1 erläuterter Aspiration, ein

Abführen von Flüssigkeit aus dem Flüssigkeitsbereich 10, hin zu einem Auslass 70 der Kassette 50, an welchen ein Wegwerf-Abfallbeutel 33 angebracht werden kann. Somit kann der Flüssigkeitsbereich 10 wieder entleert werden, und die Kassette 50 braucht bei vollem Flüssigkeitsbereich 10 nicht zwingend gleich ausgetauscht zu werden. Weitere und detailliertere erfindungsgemäße Anwendungsmöglichkeiten dieser zusätzlichen Pumpeinrichtung 60 werden im Folgenden beschrieben. Gezeigt ist auch ein optionaler zweiter Aspirationsanschluss 12b und dafür funktional nötige Ventile 65c und 65d. Diese Ausführungsform einer Kassette 50 kann beispielsweise auch gebildet werden, indem eine Basis-Kassette, welche bereits die zusätzliche Pumpeinrichtung 60 aufweist, bei Bedarf mit einem erfindungsgemäßen Clean-Venturi Wechselteil ergänzt wird, welches hier beispielsweise mit dem Bereich 36 bezeichnet ist. Die BasisKassette kann im speziellen wie in EP 3 318 290 oder EP 3 318 291 vom selben Tag und Anmelder, welche hiermit per Referenz mit einbezogen wird, ausgebildet sein.

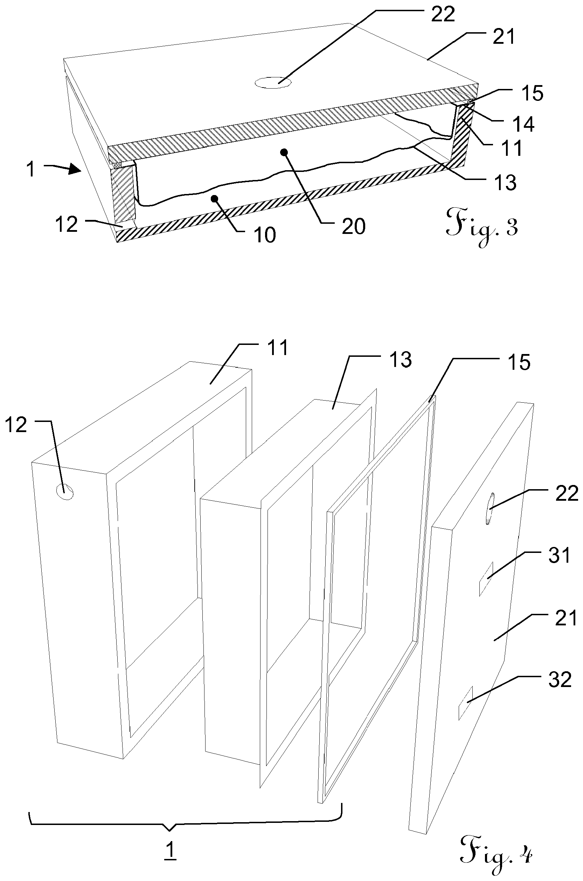

**Fig. 3** zeigt eine beispielhafte, vereinfachte Illustration eines erfindungsgemäßen Wechselteils 1 in einer Schnittdarstellung. Darin gezeigt ist die Wanne 11 oder Halbschale 11, welche rigide, z.B. aus einem rigiden Material wie beispielsweise einem oben genannten Hartkunststoff oder einem Metall ausgebildet ist. Ein mögliches Beispiel eines konkret verwendbaren Materials für eine Ausführungsform wäre etwa ein für medizinische Anwendungen zugelassenes Polypropylen oder ein anderes vergleichbares Hartkunststoff-Material, welches die für diese Anwendung wesentlichen Eigenschaften erfüllt, insbesondere z.B. mit einem Biegemodul von z.B. etwa 800 MPa oder mehr und einem Zugmodul (1 mm/min) von z.B. etwa 800 MPa oder mehr. Vorzugsweise kann diese Wanne 11 z.B. mittels Spritzgießen oder Tiefziehen hergestellt werden. Die Halbschale 11 bildet dabei einen Teil des Flüssigkeitsbereichs 10 aus, welcher über den Anschluss 12 mit dem ophthalmologischen Aspirationssystem verbindbar ist. Mit der Halbschale 11 in hermetisch abgedichteter Weise verbunden ist die Membrane 13, welche auf einer inneren ihrer Flächenseiten einen weiteren Teil des Flüssigkeitsbereichs 10 ausbildet. Die Membrane 13 ist dabei insbesondere als Folie ausgebildet. Beispielsweise ist diese aus einem der oben genannten Kunststoffe und/oder mit einer Metallfolie derart ausgebildet, dass sie flexibel ist, speziell indem sie eine entsprechende, und dem Material angepasste, geringe Dicke oder Wandstärke aufweist. Vorzugsweise eine Dicke von unter 1 mm, beispielsweise eine Dicke von 0,06 mm bei der Verwendung von Polyamid (PA) und/oder Polypropylen (PP). In einer Ausführungsform kann die Folie auch aus mehreren Materialen ausgebildet sein, beispielsweise mit einer Schicht aus PA von ca. 20 µm Dicke und einer Schicht aus PP von ca. 50 µm Dicke. Damit können die Vorzuge der Materialen kombiniert werden, beispielsweise kann die PP-Schicht vorteile bezüglich der Verschweißbarkeit mit der Halbschale 11 aufweisen, insbesondere wenn letztere ebenfalls aus PP ausgebildet ist. Entsprechend kann die Folie 13 also auf der Innenseite des Flüssigkeitsbereichs 10 aus demselben Material wie die Halbschale 11 oder einem kompatiblen Material aufgebaut sein. Die Außenseite der Membrane 13 kann beispielsweise aus einem Material mit anderen Vorzügen bestehen, beispielsweise bezüglich Reißfestigkeit, Bariereigenschaften, Dichtungseigenschaften, Flexibilität, etc. Im oben genannten Beispiel etwa aus PA, aber auch aus anderen Materialen, auch etwa Aluminium.

Das hermetische Versiegeln von Halbschale 11 und Membrane 13 kann beispielsweise durch Verschweißen, Ultraschallschweißen, Verkleben, Verpressen, Klemmen, durch Einlegen der Membrane 13 in eine Spritzgussform, Mehrkomponenten-Spritzguss, etc. erfolgen. Im gezeigten Beispiel erfolgt dies auf der Siegelschulter 14.

Auf der, dem Flüssigkeitsbereich 10 gegenüberliegenden, äußeren Seite der Membrane 13, liegt der Unterdruckbereich 20. Dieser ist, bei nicht eingelegtem Wechselteil 1 zumindest teilweise offen, sodass von dieser Seite (zumindest ein pneumatischer) Zugang zu der Membrane 13 besteht. Insbesondere kann in einer Ausführungsform die Membrane 13 des Wechselteils 1 in nicht eingelegtem Zustand offen zugänglich sein, alternativ kann in einer anderen Ausführungsform der Wechselteil 1 aber auch im Unterdruckbereich 20 einen Deckel über der Membrane 13 aufweisen, welcher den Unterdruckbereich 20 bis auf einen Luft-Anschluss 22 zur Einbringung des Unterdruckes hermetisch abschließt. Insbesondere bei der Ausführungsform ohne Deckel am Wechselteil 1, wird ein Teil des Unterdruckbereichs 20 durch ein sich im Gerät befindliches Gegenstück 21 gebildet. Dieses Gegenstück 21 ist mit einer sich ebenfalls im Gerät befindlichen (hier nicht gezeigten) Einrichtung zur Erzeugung eines Unterdrucks in pneumatischer Verbindung. In der hier gezeigten, vereinfachten Skizze ist dieses Gegenstück 21 als Platte dargestellt, welche - bei im Gerät eingelegtem Wechselteil 1 - über einer dazwischenliegenden Dichtung 15 auf der Unterdruckseite des Wechselteils zur Anlage gebracht wird. Allenfalls abgesehen von der Einrichtung zur Erzeugung des Unterdrucks, wird damit ein, insbesondere hermetisch, abgeschlossenes Volumen als Unterdruckbereich 20 gebildet, welches zur Aspiration mit dem Unterdruck beaufschlagbar ist.

In einer anderen erfindungsgemäßen Ausführungsform, kann das Gegenstück 21 - unter Erfüllung der zuvor genannten Funktion - von der hier gezeigten Plattenform abweichen, beispielsweise als, Kavität, Halbschale, etc. ausgebildet sein. Die Dichtung 15 kann dabei dem Wechselteil 1 oder dem Gegenstück 21 oder auch beiden zugeordnet sein. Vorzugsweise ist die Dichtung 15 dem Wechselteil 1 zugeordnet, da diese dann mit dem Wechselteil 1 ausgetauscht wird, und somit nicht auf Dauer verschleißen kann.

In einer anderen erfindungsgemäßen Ausführungsform kann auch das Gegenstück 21 als Halbschale oder Kavität ausgebildet sein, und das Wechselteil 1 substantiell flach oder ebenfalls als Halbschale. Eine praktische Ausführungsform könnte also beispielsweise auch vice-versa aufgebaut sein. Beispielsweise etwa mit einer Kavität im Gerät 99, welche beim Einlegen des Wechseleinsatzes 1 über einen, von der Membrane 13 in Form eines flexiblen Beutels gebildeten Flüssigkeitsbereich 10 des Wechseleinsatzes 1 hermetisch gegenüber der Raumluft abdichtend zum Anliegen kommt. Der den Flüssigkeitsbereich 10 bildende Beutel kommt dabei im Unterdruckbereich 20, der als Unterdruckkammer oder Saugkammer von der Kavität im Gerät 99 gebildet wird, zu liegen. Dabei kann dieser Beutel auch zumindest Teilweise mit dem Wechselteil 1 verbunden oder von diesem gebildet sein.

**Fig. 4** zeigt beispielhaft eine vereinfachte Explosions-darstellung eines Wechselteils 1 und eines geräteseitigen Gegenstücks 21, wobei nur die funktional wesentlichsten Elemente dargestellt sind. Die feste Wanne 11 bildet einen Träger des Wechselteils 1 aus, welcher gehandhabt und in ein ophthalmologisches Gerät eingelegt wird. Mit der Wanne 11 umlaufend fest und insbesondere Luft- und Feuchtigkeitsdicht verbunden ist die flexible Folie 13. Auch die Dichtung 15 ist vorzugsweise Teil des Wechselteils 1 und mit diesem insbesondere fest verbunden. Die Dichtung ist derart angeordnet, dass sie auf der Unterdruckseite der Folie 13 umschließt, insbesondere derart, dass wechselteilseitig über die Dichtung ein hermetisch abgeschlossener Unterdruckbereich ausgebildet werden kann.

Das Einlegen kann z.B. in der gezeigten Hochkantausrichtung, also z.B. in der Darstellung nach hinten, in einem Schlitz im Gerät erfolgen, in welchem das Wechselteil 1 dann daraufhin von der Seite, also z.B. in dieser Darstellung von der rechten Seite, her mit dem geräteseitigen Gegenstück 21 im Gerät derart geklemmt wird, dass die Dichtung 15 ihre Funktion erfüllt. In einer alternativen Ausführungsform könnte das Wechselteil 1 auch flach mit der Membran-Seite zum Gerät hin eingelegt werden, sodass die Dichtung 15 mit dem geräteseitigen Gegenstück 21 abschließt - in anderen Worten, in der gezeigten Darstellung das Gerät also rechts angeordnet ist, wobei hier nur das Gegenstück 21 als Teil des Geräts dargestellt ist. Optional kann das Gerät auch einen hier gezeigten Füllstandssensor 31 für den Flüssigkeitsbereich und/oder einen Feuchtigkeitssensor 32 zur Leckdetektierung aufweisen, vorzugsweise im Unterdruckbereich.

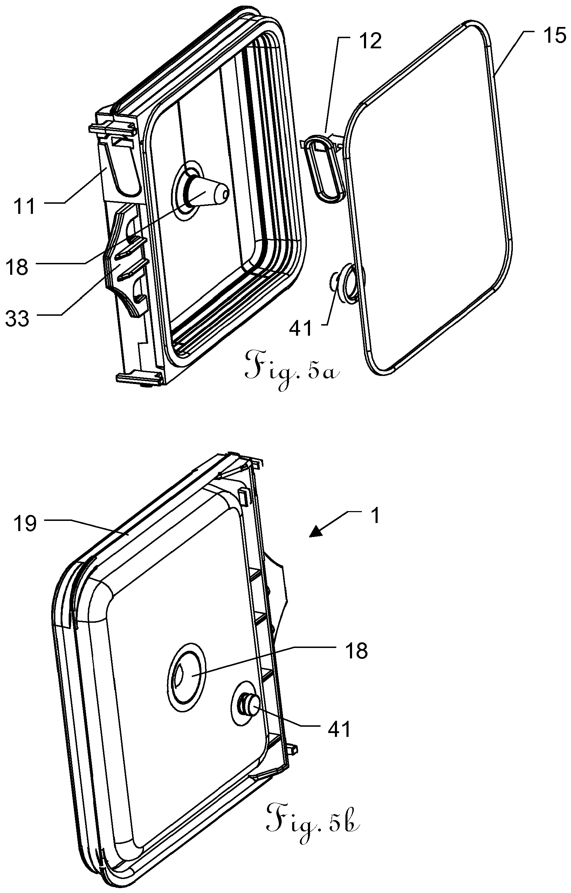

**Fig. 5a** stellt eine mögliche konstruktive Ausführungsform eines erfindungsgemäßen Wechselteils 1 dar. Die Wanne 11 ist als Spritzgussteil aus einem Hartkunststoff dargestellt. Zumindest Teile des inneren Hohlraums dieser Wanne 11 bilden dabei, gemeinsam mit der Membrane 13 den Flüssigkeitsbereich 10 aus. Das Wechselteil weist außen eine Lasche 33 zur Handhabung und/oder zur Befestigung an einer Basis-Kassette auf. In der Mitte der Wanne 11 ist in dieser Ausführungsform eine domförmige Wölbung 18 ausgebildet, mittels welcher beispielsweise eine bessere Formstabilität und/oder für die hier nicht gezeigten Membrane 13 ein vorteilhaftes Verformungsverhalten bei Volumenänderungen des Flüssigkeitsbereichs erzielt werden kann. Der Übersichtlichkeit wegen abgesetzt davon gezeigt sind die, vorzugsweise aus einem Elastomer oder thermoplastischen Elastomer ausgebildeten, Dichtelemente für den Aspirationsanschluss 12, sowie für das hier nicht gezeigte Gegenstück 21, sowie eine Messmembrane 41 für eine Druckmesseinrichtung im Flüssigkeitsbereich. Ein Beispiel eines konkreten Materials für eine mögliche Ausführungsform wäre etwa ein für medizinische Anwendungen zugelassenes thermoplastisches Elastomer oder Elastoplast, oder ein anderes in seinen für diese Anwendung wesentlichen Eigenschaften vergleichbares Weichkunststoff-Material, insbesondere z.B. mit einer ShoreA-Härte von etwa 20 bis 100.

**Fig. 5b** zeigt die obige beispielhafte Ausführungsform des erfindungsgemäßen Wechselteils 1 von der anderen Seite. Die Wölbung 18 bildet hier entsprechend eine Einbuchtung, welche beispielsweise zur Zentrierung des Wechselteils 1 im Gerät genutzt werden kann. Oben und unten befindet sich in diesem Beispiel auch ein Führungssteg 19, um das Einführen des Wechseleinsatzes 1 in das Gerät zu erleichtern. Gezeigt ist auch der von Außen zugängliche Teil 41 für die Druckmesseinrichtung.

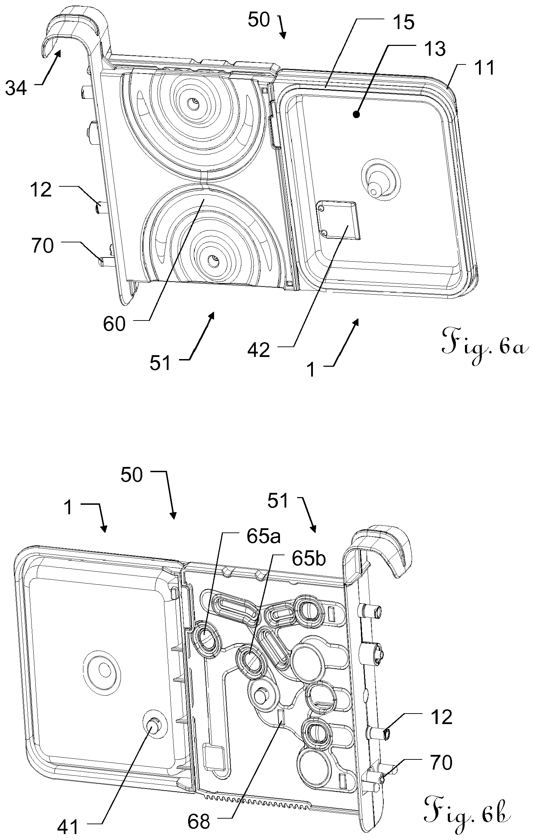

In **Fig. 6a** ist eine weitere beispielhafte Ausführungsform eines erfindungsgemäßen Wechsel-Einsatzes 1 gezeigt, weleher als Teil einer im gesamten auswechselbaren ophthalmologischen Kassette 50, also speziell in Verbindung mit einer Basis-Kassette 51 mit weiteren Funktionalitäten ausgebildet ist. In der Ausführungsform zuvor war der Wechsel-Einsatz 1 an sich als derartige ophthalmologischen Kassette 50 ausgebildet, welche jedoch weniger Funktionalität aufwies als das nun gezeigte Beispiel.

Die Halbschale 11 oder Venturi-Wanne 11, Dichtung 15 sowie Folie 13 als Trennung von Flüssigkeitsbereich 10 und Unterdruckbereich 20 wurden bereits beschrieben und können hier analog oder ähnlich ausgeführt sein. Hier dargestellt ist eine feste Abdeckung 42 über der Druckmessmembrane 41 im Flüssigkeitsbereich 10, um eine Berührung mit der Membrane 13 der Venturi-Wanne 11 zu vermeiden, da dies eine Druckmessung behindern könnte. Der Flüssigkeitsbereich 10 der Venturi-Wanne 11 steht in hydraulischer Verbindung mit dem Aspirationsanschluss 12 an der, in eingelegtem Zustand zugänglichen Front der Kassette 50. Zum insbesondere einhändigen Einlegen ist dabei der Haltebügel oder Handgriff 34 ausgebildet. Hier zusätzlich gezeigt ist eine optionale zusätzliche Pumpeinrichtung 60, welche im gezeigten Beispiel als Peristaltikpumpe ausgebildet ist, aber beispielsweise auch eine andere Pumpeinrichtung wie eine Membranpumpe sein kann. Speziell bezeichnet ist ein flexibler Flüssigkeitskanalabschnitt in der Kassette 50, mittels welchem durch (hier nicht gezeigte) Roller im Gerät eine Quetschpumpwirkung erzielbar ist. Diese zusätzliche Pumpeinrichtung steht in hydraulischer Verbindung mit dem Aspirationsflüssigkeitssystem, also insbesondere mit dem Flüssigkeitsbereich 10. Als ein weiterer erfindungsgemäßer Teilaspekt ist mit dieser zusätzlichen Pumpeinrichtung 60 ein Fördern von Flüssigkeit (oder auch von allfälliger Luft) aus dem Flüssigkeitsbereich zu einem Abfallanschluss durchführbar. An diesem Abfallanschluss ist z.B. ein Beutel zur Aufnahme der aspirierten Flüssigkeit anschließbar, welcher Beutel dann entsorgt werden kann. Mit diesem Aspekt kann ein Füllstand im Flüssigkeitsbereich 10 reduziert werden, vorzugsweise kann der gesamte Flüssigkeitsbereich 10 zumindest annährend entleert werden. Dies kann erfindungsgemäß während der Operation, in kurzen Operationspausen oder außerhalb der Operationszeit stattfinden. Auch kann diese zusätzliche Pumpeinrichtung 60 als Rückfalloption bei Versagen des erfindungsgemäßen Clean-Venturi-Systems genutzt werden. Auch ist erfindungsgemäß eine Mischanwendung von Clean-Venturi-System und zusätzlicher Pumpeinrichtung 60 möglich, insbesondere mit fließendem oder abruptem Übergang oder als dauerhafte Mischanwendung. Auch kann die zusätzliche Pumpeinrichtung 60 als zusätzlicher Aspirationsdruck- oder Aspirationsvolumen-Boost genützt werden um, beispielsweise bei Okklusionen eine Aspirationsfördererhöhung zu bewirken. Auch kann mit der zusätzlichen Pumpeinrichtung 60 eine kurzzeitige Rückförderung von Flüssigkeit zur Lösung von Okklusionen genutzt werden. Ein vollständiges entleeren des Flüssigkeitsbereichs 10 kann beispielsweise auch dadurch detektiert werden, dass dann der Unterdruck im Aspirationssystem (z.B. detektiert über die Druckmesseinrichtung 40) rapide ansteigt. Um für ein erneutes Aktivieren der Aspiration vom Patient einen sanften Druckanstieg zu gewährleisten, kann ein kurzes Rückfördern der Entleerungseinrichtung zum Abbau dieses erhöhten Unterdrucks angewandt werden.

**Fig. 6b** stellt obige, beispielhafte Ausführungsform von der anderen Seite her gesehen dar. Die bezeichneten Elemente wurden zuvor schon erläutert. Zusätzlich gezeigt sind die Ventile 65a oder 65b im Aspirationsflüssigkeitspfad, mit welchen ein Durchflussquerschnitt variierbar, insbesondere verschließ- und freigebbar, ist. Mittels diesen kann die Aspiration in den Flüssigkeitsbereich geschaltet oder geregelt werden. Auch eine Zu- oder Umschaltung bezüglich der zusätzlichen Pumpeinrichtung 60 ist mit diesen durchführbar. Gezeigt ist auch eine Luftblasenüberwachung 68, hier ausgebildet mit einem Sichtfenster in der Kassette 50 zur optischen Detektierung von Luft im Aspirationssystem.

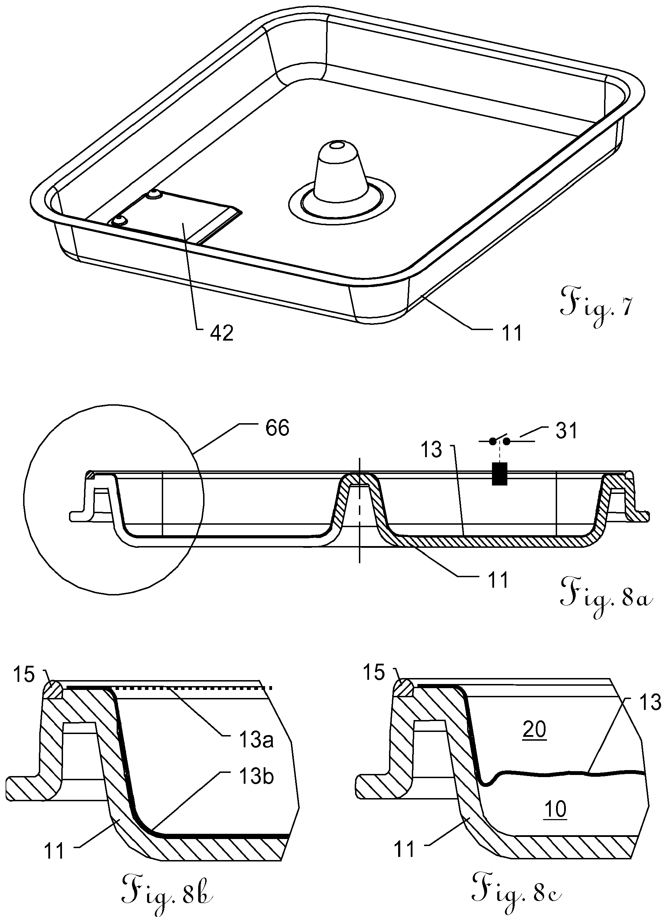

**Fig. 7** zeigt ein Beispiel einer Ausführungsform einer erfindungsgemäßen Venturi-Wanne 11 als ein Kernstück eines erfindungsgemäßen Wechsel-Einsatzes 1. Zu sehen ist darin auch eine beispielhafte Drucksensorabdeckung 42 als eine Ausführungsform zur Bereitstellung einer Druckmesseinrichtung, mittels welcher beispielsweise bei eine vollständige Leerung des Flüssigkeitsbereichs 10 durch eine zusätzliche Pumpeinrichtung 60 anhand eines Anstiegs des erfassten Unterdruckwertes im Flüssigkeitsbereich 10 feststellbar ist, und/oder mittels welchem eine Fehlfunktion oder ein voller Flüssigkeitsbereich anhand eines Abfallens des Unterdruckwerts im Flüssigkeitsbereich 10 feststellbar ist.

**Fig. 8a** illustriert nun eine erfindungsgemäß ein Beispiel einer Ausführungsform einer erfindungsgemäßen Venturi-Wanne 11 als ein Kernstück eines erfindungsgemäßen Wechsel-Einsatzes 1 in einer Schnittdarstellung. Darin ist nun auch die als Folie ausgeführte, dünne Membrane 13 in Ihrer Lage bei leerem Flüssigkeitsbereich 10 gezeigt. Das Schaltersymbol 31 symbolisiert eine geräteseitige Füllstandsüberwachung des Flüssigkeitsbereichs 10, was beispielsweise durch einen von der Membrane 13 betätigtes Schaltelement erfolgen kann. Das Detail 66 ist nochmals in **Fig. 8b** dargestellt. Dabei kann die Membrane in Stellung 13a die Ausgangslage in einem ersten Produktionsschritt des Wechsel-Einsatzes darstellen. In einem darauf folgenden Produktionsschritt kann dann die Membrane 13 von Stellung 13a zu Stellung 13b tiefgezogen werden, vorzugsweise durch Einbringen eines Unterdrucks in den Flüssigkeitsbereich 10 über dessen Aspirationsanschluss, optional auch mit einer Erwärmung der Membrane 13. Insbesondere kann dabei dann die Membrane 13 in Lage 13b als Ruheposition verbleiben und der Wechseleinsatz in dieser Form zur erstmaligen Verwendung bereitgestellt werden. Alternativ könnte die Membrane 13 auch in einer anderen Position bereitgestellt werden, und erst im Gerät bei deren Inbetriebnahme, beispielsweise mittels der weiteren Pumpeinrichtung 60, in die Lage 13b gebracht werden, was aber beispielsweise bezüglich des Zeitaufwands meist unvorteilhaft wäre.

**Fig. 8c** zeigt nochmals Detail 66 aus Fig. 8a, jedoch mit der Membrane 13 in einer Zwischenstellung, beispielsweise während einer Aspiration, bei welcher sowohl Flüssigkeitsbereich 10 und Unterdruckbereich 20 ein Volumen zwischen ihren Maximal- und Minimalwerten haben.

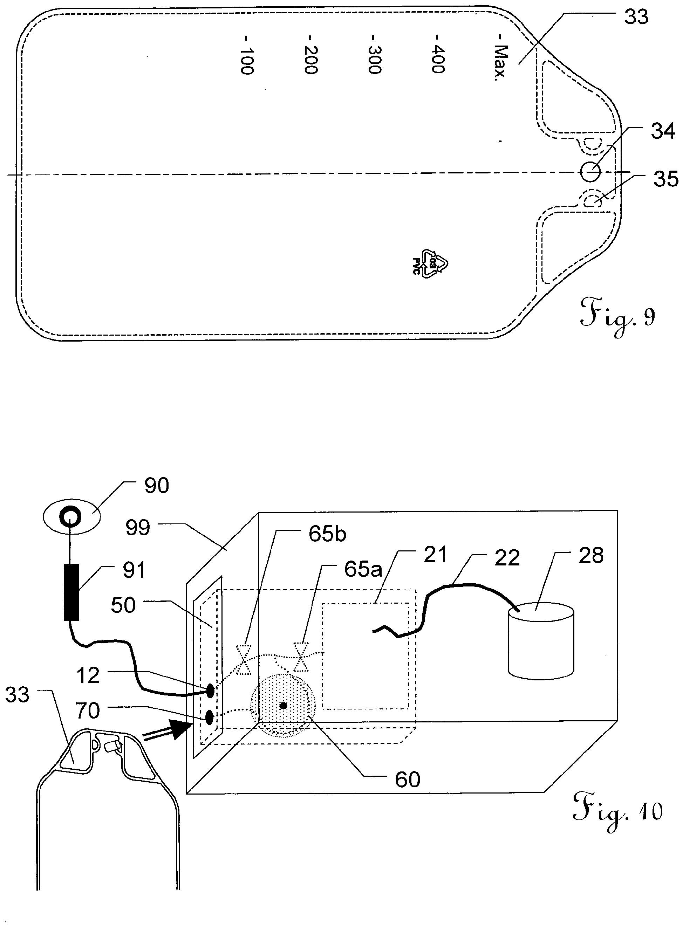

**Fig. 9** zeigt eine beispielhafte Ausführungsform eines Abfallbehälters 33, beispielsweise eines Abfallsacks oder Abfallbeutels. Mit seinem Anschluss 34 ist er mit dem Abfallanschluss 70 der Kassette verbindbar, speziell ist dieser Abfallanschluss 70 gegen Vertauschung mit einem anderen Anschluss der Kassette mechanisch und/oder optisch Codiert. In den Abfallbehälter 33 kann entsprechend eines Aspekts der Erfindung Flüssigkeit aus dem Flüssigkeitsbereich 10 der Clean-Venturi-Wanne 10 abführbar sein. Speziell kann der Abfallbehälters 33 eine spezielle Vorrichtung 35 zum direkten Einhängen an der Kassette 50 aufweisen, und die Kassette 50 das entsprechende Pendant dazu.

**Fig. 10** zeigt beispielhaft einen Ausschnitt einer beispielhaften Ausführungsform eines erfindungsgemäßen ophthalmologischen Geräts 99 mit einer darin aufgenommenen beispielhaften Ausführungsform einer erfindungsgemäßen ophthalmologischen Kassette 50 als einer Ausführungsform eines erfindungsgemäßen Wechsel-Einsatzes 1. Für Details der Kassette 50 bzw. des Wechsel-Einsatzes 1 wird auf obige Beschreibung verwiesen. An dessen Aspirationsanschluss 12 ist beispielsweise ein Phako-Handstück 91 angeschlossen, mit welchem ein Eingriff am Auge 90 vorgenommen werden kann. In der Kassette 50 ist dieser Aspirationsanschluss 12 wie beschrieben, insbesondere beispielsweise über zumindest einen Teil von Ventilen, Drucksensoren, Luftblasendetektoren, zusätzlichen Pumpeinrichtungen, etc., mit dem Flüssigkeitsbereich des Wechsel-Einsatzes (resp. der Kassette) verbunden - was in diesem Beispiel nur symbolhaft in seiner Grundstruktur dargestellt ist und insbesondere nach Fig. 1 und Fig. 2 und der zugehörigen Beschreibung ausgeführt sein kann. Mit der in dieser Ausführungsform gezeigten, zusätzlichen Pumpeinrichtung 60 kann auch Flüssigkeit vom Flüssigkeitsbereich 10 in den direkt am Abfallanschluss 70 angebrachten Abfallbeutel 33 abgeführt werden. Durch die Membrane 13 vom Flüssigkeitsbereich 10 hermetisch getrennt liegt der Unterdruckbereich 20. Dieser Unterdruckbereich 20 wird in dieser Ausführungsform von der Kassette 50 und dem Gegenstück 21 gebildet, und aus diesem Unterdruckbereich 20 kann Luft abgesaugt werden, wie gezeigt etwa indem das Gegenstück 21 über den Anschluss 22 mit einer Vakuumpumpe 28 verbunden ist. Der Übersichtlichkeit halber sind weiter Details des Geräts hier nicht gezeigt.

## Patentansprüche

1. Wechsel-Einsatz (1) für ein ophthalmologisches Medizinalgerät (99) zur Aspiration von Flüssigkeit
- insbesondere welche Flüssigkeit im Rahmen einer Saugspülung bei einem ophthalmo-chirurgischen Eingriff abzuführen ist -
mit:
o einem Flüssigkeitsbereich (10) zur Aufnahme der abzusaugenden Flüssigkeit und
∘ einem Unterdruckbereich (20), welcher mit einem Unterdruck beaufschlagbar ist,
wobei der Flüssigkeitsbereich (10) und der Unterdruckbereich (20) mit einer zumindest partiell flexiblen Membrane (13) hermetisch voneinander getrennt sind,
und wobei der Wechsel-Einsatz (1) derart ausgebildet ist, um mittels des Unterdrucks die Flüssigkeit in den, in seinem Volumen durch zumindest partielle Verformung der Membrane veränderbaren Flüssigkeitsbereich (10) abzusaugen.

2. Wechsel-Einsatz (1) nach Anspruch 1, **dadurch gekennzeichnet, dass**
die flexible Membrane (13) derart ausgebildet ist, dass mittels der flexiblen Membrane (13) eine Übertragung des pneumatischen Unterdrucks im Unterdruckbereich (20) auf einen hydraulischen Unterdruck im Flüssigkeitsbereich (10) erfolgt, und wobei der Wechsel-Einsatz (1) mit einer Halbschale (11) ausgebildet ist, mit welcher die Membrane (13) einen in seinem Volumen variablen Hohlraum ausbildet, welcher zumindest einen Teil des Flüssigkeitsbereichs ausbildet und
ein Volumen von zumindest annähernd Null bis hin zu einigen zig oder hundert Millilitern aufweist.

3. Wechsel-Einsatz (1) nach Anspruch 1 oder 2, **dadurch gekennzeichnet, dass**
der Wechsel-Einsatz (1) mit einer Halbschale (11) - insbesondere aus einem Thermoplast wie einem Polypropylen oder Polyethylen - ausgebildet ist in welcher die Membrane (13) - insbesondere aus einem Polymer wie etwa einem Polyamid und/oder Polypropylen - als eine flexible Folie derart angeordnet ist, dass zwischen der Halbschale (11) und einer Innenseite der Membrane (13) eine Saugkammer (10) ausgebildet wird, und an einer Außenseite der Membrane (13) der Unterdruckbereich (20) ausgebildet wird
- insbesondere wobei eine Schulter der Halbschale (11) bei eingelegtem Wechsel-Einsatz (1) mit einem Gegenstück (21) in dem Medizinalgerät (99) eine umlaufend abgedichtete Unterdruckkammer mit einer Luft-Absaugung zur Bereitstellung des Unterdrucks bildet, speziell wobei beim Einlegen des Wechsel-Einsatzes (1) eine Klemmkraft zwischen dem Gegenstück (21) und dem Wechsel-Einsatz (1) aufgebracht wird.

4. Wechsel-Einsatz (1) nach zumindest einem der Ansprüche 1 oder 2 oder 3, **dadurch gekennzeichnet, dass** der Wechsel-Einsatz (1) als austauschbare Kassette (50) für das ophthalmologisches Medizinalgerät (99) ausgebildet ist, wobei
der Flüssigkeitsbereich (10) eine Saugkammer zur zumindest temporären Aufnahme von aspirierter Flüssigkeit ausbildet und mit einem Aspirationsanschluss (12) eines chirurgischen Operations-Bestecks (91) hydrostatisch verbindbar ist, und der Flüssigkeitsbereich (10) mit
▪ der flexiblen Membrane (13), welche vorzugsweise derart großflächig ausgebildet ist, dass eine Fläche der Membrane (13) im Verhältnis zur gesamten Oberfläche des Flüssigkeitsbereichs (10) mehr als 16%, insbesondere mehr als 25% oder mehr als 35%, ausmacht, und
▪ einer festen Halbschale (11) ausgebildet ist, wobei die Membrane (13), wenn die Kassette (50) im Medizinalgerät (99) eingelegt ist, von außen über den Unterdruckbereich (20) mit einem Unterdruck beaufschlagbar ist, sodass eine Unterdruckübertragung via der Membrane (13) bewirkbar ist.

5. Wechsel-Einsatz (1) nach zumindest einem der Ansprüche 1 oder 2 oder 3, **dadurch gekennzeichnet, dass** der Wechsel-Einsatz (1) als optionales Zusatzmodul ausgebildet ist, welches an eine ophthalmologische Basis-Kassette (51), welche bereits zumindest eine Peristaltikpumpe (60) aufweist, anbringbar ist,
- insbesondere wobei das Zusatzmodul und die Basis-Kassette (51) miteinander verschnappbar sind und bei der Anbringung eine hydraulische Verbindung des Flüssigkeitsbereichs (10) des Wechsel-Einsatzes (1) mit einem Aspirationssystem der Basis-Kassette (51) herstellbar ist.

6. Wechsel-Einsatz (1) nach zumindest einem der Ansprüche 1 bis 5, **dadurch gekennzeichnet, dass** der Unterdruck von einer Luft-Absaug-Einrichtung (28) im Medizinalgerät (99) erzeugt wird, insbesondere von einer elektrisch betriebenen Teil-Vakuum-Pumpe oder einer Venturi-Düse, und wobei ein gewünschter Wert des von der Luft-Absaug-Einrichtung (28) erzeugten Unterdrucks zeitlich variabel vorgebbar ist.

7. Wechsel-Einsatz (1) nach zumindest einem der Ansprüche 1 bis 6, **dadurch gekennzeichnet, dass** der Flüssigkeitsbereich (10) eine Druckerfassungseinrichtung (40) aufweist oder mit einer derartigen in hydrostatischer Verbindung steht, mit welcher ein Unterdruck in dem Flüssigkeitsbereich (10) erfassbar ist.

8. Wechsel-Einsatz (1) nach zumindest einem der Ansprüche 1 bis 7, **dadurch gekennzeichnet, dass** beim Wechsel-Einsatz (1) in einem Flüssigkeitspfad zwischen dem Flüssigkeitsbereich (10) und einem Anschluss (12) für eine Aspirationsleitung (12) ein Ventil (65) vorgesehen ist, mit welchem ein Querschnitt des Flüssigkeitspfads veränderbar ist, speziell freigegeben oder verschlossen werden kann.

9. Wechsel-Einsatz (1) nach zumindest einem der Ansprüche 1 bis 8, **dadurch gekennzeichnet, dass** der Wechsel-Einsatz (1) eine Pumpvorrichtung (60) - insbesondere eine Peristaltikpumpe - aufweist, mit welcher Flüssigkeit aus dem Flüssigkeitsbereich (10) in einen austauschbaren Abfallbehälter (33) abführbar ist, sodass mit der Pumpvorrichtung (60) ein Entleeren des Flüssigkeitsbereichs (10) durchführbar ist, vorzugsweise wobei hierzu der Aspirationsanschluss (12) mittels eines dafür vorgesehenen Ventils (65b) vom Flüssigkeitsbereich (10) abtrennbar ist.

10. Wechsel-Einsatz (1) nach zumindest einem der Ansprüche 1 bis 9, **dadurch gekennzeichnet, dass** im Wechsel-Einsatz (1) Flüssigkeitspfade derart ausgebildet sind, dass - insbesondere mittels zumindest eines dafür vorgesehenen Ventils (65,65a) - eine Umschaltung zwischen einer Aspiration mittels zumindest zwei von:
▪ des auf den Flüssigkeitsbereich (10) übertragenen Unterdrucks, oder
▪ einer alternativen Pumpvorrichtung (60), insbesondere einer Peristaltikpumpe, oder
▪ einer Kombination dieser beiden
durchführbar ist,
- insbesondere wobei hierbei als die alternative Pumpvorrichtung (60) dieselbe Pumpvorrichtung (60) nach Anspruch 7 genutzt wird, mit welcher auch Flüssigkeit in den Abfallbehälter (33) abführbar ist.

11. Wechsel-Einsatz (1) nach zumindest einem der Ansprüche 1 bis 10, **dadurch gekennzeichnet, dass** der Wechsel-Einsatz (1) einen Füllstands-Sensor des Flüssigkeitsbereichs (10) aufweist
- insbesondere ausgebildet als ein Drucksensor (40) und/oder als ein Sensor (31) zur Bestimmung einer Lage der Membrane (13) - und/oder
der Wechsel-Einsatz (1) einen Flüssigkeits- oder Feuchtigkeits-Sensor (32) im Unterdruckbereich (20) aufweist - insbesondere ausgebildet zur Detektierung von Defekten der Membrane (13).

12. Gerät (50) zur Augenoperation mit
einer Aufnahmevorrichtung für einen Wechsel-Einsatz (1) nach zumindest einem der Ansprüche 1 bis 11,
wobei das Gerät (99) eine Luft-Absaug-Einrichtung (28) zur Abpumpung von Luft aus dem, vom Flüssigkeitsbereich (10) des Wechsel-Einsatzes (1) hermetisch getrennten, Unterdruckbereich (20) des Wechsel-Einsatzes (1) aufweist, insbesondere wobei die Luft-Absaug-Einrichtung (28) mit einer Teil-Vakuumpumpe oder einer Venturi-Düse ausgebildet ist, insbesondere wobei die Aufnahmevorrichtung für den Wechsel-Einsatz (1) derart ausgebildet ist, dass ein hermetisch abschließendes, lösbares Fügen eines umlaufenden Bereichs des Wechsel-Einsatz (1) mit einem Gegenstück (21) des Geräts (99) zur Bildung des geschlossenen Unterdruckbereichs (20) erfolgt.

13. Verfahren zur Aspiration einer Flüssigkeit, durchgeführt in einem ophthalmologischen Gerät (99), insbesondere eine Aspiration nach einem Venturi-Prinzip mittels pneumatischer Unterdruckabsaugung, mit:
▪ einem Bereitstellen eines hermetisch abgeschlossenen Flüssigkeitsbereichs (10), welcher zumindest teilweise von einer flexiblen Membrane (13) gebildet wird, in einem Wechsel-Einsatz (1) für das ophthalmologisches Gerät (99),
welcher mit einem Aspirationsanschluss (12) in hydraulische Verbindung bringbar ist,
▪ einem Absaugen von Luft an einer, gegenüber dem Flüssigkeitsbereich (10) auf einer Außenseite der Membrane (13) liegenden Seite, welche Außenseite Teil eines Unterdruckbereichs (20) ist,
▪ wobei durch die Membrane (13) ein hermetisches abtrennen des Flüssigkeitsbereichs (10) vom Unterdruckbereich (20) sowie von der Raumluft erfolgt,
▪ insbesondere wobei mittels der Membrane (13) eine Übertragung eines pneumatischen Unterdrucks im Unterdruckbereich (20) auf einen hydraulischen Unterdruck im Flüssigkeitsbereich (10) erfolgt.

14. Verfahren nach Anspruch 13, **gekennzeichnet, durch** ein Messen eines Druckes im Flüssigkeitsbereich (10) zur Defekts- und/oder Füllstandserkennung im Flüssigkeitsbereich (10) und/oder ein Detektieren von Feuchtigkeit oder Flüssigkeit im Unterdruckbereich (20) zur Defekterkennung der Membrane (13) erfolgt.

15. Verfahren nach Anspruch 13 oder 14, **dadurch gekennzeichnet, dass**
ein Umschalten zwischen einer Aspiration:
▪ mittels des pneumatischen Unterdrucks, welcher via der Membrane (13) auf den Flüssigkeitsbereich (10) wirkt und/oder
▪ mittels einer hydraulischen Volumenförderung (60), insbesondere mit einer Peristaltikpumpe, durchführbar ist.

16. Verfahren nach einem der Ansprüche 13 bis 15, **dadurch gekennzeichnet, dass**
ein Abführen von Flüssigkeit aus dem Flüssigkeitsbereich (10) in einen Abfallbehälter (33), insbesondere durch peristaltisches Pumpen, durchführbar ist, insbesondere wobei ein Entleeren des Flüssigkeitsbereichs (10) während Aspirationspausen erfolgt, vorzugsweise automatisch und/oder fortlaufend.

## Claims

1. A replacement insert (1) for an ophthalmological medical device (99) for the aspiration of liquid, - in particular which liquid is to be discharged within the scope of a suction flushing during an ophthalmic surgical intervention - comprising:
a liquid region (10) for receiving the liquid to be suctioned away and
a negative pressure region (20), to which a negative pressure can be applied,
wherein the liquid region (10) and the negative pressure region (20) are hermetically separated from one another by means of an at least partially flexible membrane (13),
and wherein the replacement insert (1) is designed in such a way as to suction the liquid into the liquid region (10) by means of the negative pressure.

2. The replacement insert (1) according to claim 1, **characterized in that** the flexible membrane (13) is designed in such a way that the pneumatic negative pressure in the negative pressure region (20) is transferred by means of the flexible membrane (13) to a hydraulic negative pressure in the liquid region (10), wherein the replacement insert is designed with a half-shell (11), with which the membrane (13) forms a hollow space of variable volume, the hollow space forming at least a part of the liquid region and having a volume of from at least approximately zero to several tens or hundreds of milliliters.

3. The replacement insert (1) according to claim 1 or 2, **characterized in that** the replacement insert (1) is designed with a half-shell (11) - in particular formed from a thermoplastic, such as a polypropylene or polyethylene - in which the membrane (13) - in particular formed from a polymer, such as a polyamide and/or polypropylene - is arranged as a flexible film in such a way that a suction chamber (10) is formed between the half-shell (11) and an inner side of the membrane (13), and the negative pressure region (20) is formed on an outer side of the membrane (13), in particular wherein a shoulder of the half-shell (11), as the replacement insert (1) is inserted, together with a counter piece (21) in the medical device (99) forms a peripherally sealed negative pressure chamber with an air suction system for providing the negative pressure, especially wherein, as the replacement insert (1) is inserted, a clamping force is applied between the counter piece (21) and the replacement insert (1).

4. The replacement insert (1) according to at least one of claims 1 or 2 or 3, **characterized in that** the replacement insert (1) is designed as an interchangeable cassette (50) for the ophthalmological medical device (99), wherein the liquid region (10) forms a suction chamber for at least temporarily receiving aspirated liquid and can be connected hydrostatically to an aspiration connection (12) of a surgical instrument (91), and the liquid region (10) is designed with
the flexible membrane (13), which is preferably designed with a surface accounting for, compared to the total surface of the liquid region (10), more than 16%, in particular more than 25%, or more than 35%, and
a solid half-shell (11),
wherein the membrane (13), when the cassette (50) is inserted in the medical device (99), can be acted on from outside by a negative pressure via the negative pressure region (20), so that a negative pressure transfer via the membrane (13) can be effected.

5. The replacement insert (1) according to at least one of claims 1 or 2 or 3, **characterized in that** the replacement insert (1) is designed as an optional auxiliary module, which can be attached to an ophthalmological basic cassette (51), which already comprises at least one peristaltic pump (60), in particular wherein the auxiliary module and the basic cassette (51) can be snap-fitted together and, when the auxiliary module is attached, a hydraulic connection of the liquid region (10) of the replacement insert (1) to an aspiration system of the basic cassette (51) can be produced.

6. The replacement insert (1) according to at least one of claims 1 to 5, **characterized in that** the negative pressure is generated by an air suction arrangement (28) in the medical device (99), in particular by an electrically operated partial vacuum pump or a Venturi nozzle, wherein a desired value of the negative pressure generated by the air suction arrangement (28) can be specified in a manner variable over time.

7. The replacement insert (1) according to at least one of claims 1 to 6, **characterized in that** the liquid region (10) comprises a pressure detection arrangement (40) or is hydrostatically connected to an arrangement of this kind, by means of which a negative pressure in the liquid region (10) can be detected.

8. The replacement insert (1) according to at least one of claims 1 to 7, **characterized in that** a valve (65) is provided in the replacement insert (1) in a liquid path between the liquid region (10) and a connection (12) for an aspiration line (12), by means of which valve a cross-section of the liquid path can be varied, in particular can be released or closed.

9. The replacement insert (1) according to at least one of claims 1 to 8, **characterized in that** the replacement insert (1) comprises a pump assembly (60) - in particular a peristaltic pump - by means of which liquid can be discharged from the liquid region (10) into an interchangeable waste container (33), so as to enable emptying of the liquid region with the pump assembly, preferably wherein for this purpose the aspiration connection (12) can be separated from the liquid region (10) by means of a valve (65b) provided for this purpose, in particular wherein the liquid region (10) can be emptied by means of the pump apparatus (60).

10. The replacement insert (1) according to at least one of claims 1 to 9, **characterized in that** liquid paths are formed in the replacement insert (1) in such a way that - in particular by means of at least one valve (65, 65a) intended for this purpose - it is possible to switch between an aspiration by means of at least two of:
the negative pressure transferred to the liquid region (10), or
an alternative pump apparatus (60), in particular a peristaltic pump, or
a combination of the two,
in particular wherein here the same pump apparatus (60) as according to claim 7, by means of which liquid can also be discharged into the waste container (33), is used as the alternative pump apparatus (60).

11. The replacement insert (1) according to at least one of claims 1 to 10, **characterized in that**
the replacement insert (1) has a filling level sensor of the liquid region (10), in particular designed as a pressure sensor (40) and/or as a sensor (31) for determining the position of the membrane (13), and/or
the replacement insert (1) comprises a liquid or moisture sensor (32) in the negative pressure region (20), in particular designed to detect defects of the membrane (13).

12. A device (50) for eye surgery comprising a receiving apparatus for a replacement insert (1) according to at least one of claims 1 to 11,
wherein the device (99) comprises an air suction arrangement (28) for pumping air away from the negative pressure region (20) of the replacement insert (1) separated hermetically from the liquid region (10) of the replacement insert (1), in particular wherein the air suction arrangement (28) is formed with a partial vacuum pump or a Venturi nozzle,
in particular wherein the receiving apparatus for the replacement insert (1) is designed in such a way that the peripheral region of the replacement insert (1) is releasably joined, in a hermetically sealed manner, to a counter piece (21) of the device (99) in order to form the closed negative pressure region (20).

13. A method for aspirating a liquid, carried out in an ophthalmological device (99), in particular an aspiration in accordance with a Venturi principle by means of negative pressure suction, with:
providing a hermetically closed liquid region (10), which is formed at least in part by a flexible membrane (13), in a replacement insert (1) for the ophthalmological device (99), which can be hydraulically connected to an aspiration connection (12),
suctioning air on a side arranged opposite the liquid region (10) on an outer side of the membrane (13), which outer side is part of a negative pressure region (20),
wherein the liquid region (10) is hermetically separated by the membrane (13) from the negative pressure region (20) and from the room air,
in particular wherein a pneumatic negative pressure in the negative pressure region (20) is transferred by means of the membrane (13) to a hydraulic negative pressure in the liquid region (10).

14. The method according to claim 13, **characterized by** a step of measuring a pressure in the liquid region (10) in order to identify a defect and/or filling level in the liquid region (10) and/or a step of detecting moisture or liquid in the negative pressure region (20) in order to identify a defect of the membrane (13).

15. The method according to claim 13 or 14, **characterized in that** it is possible to switch between an aspiration:
by means of pneumatic negative pressure, which acts via the membrane (13) on the liquid region (10) and/or
by means of a hydraulic volume delivery (60), in particular by means of a peristaltic pump.

16. The method according to any one of claims 13 to 15, **characterized in that** liquid can be discharged from the liquid region (10) into a waste container (33), in particular by peristaltic pumping, in particular wherein the liquid region (10) is emptied during aspiration pauses, preferably automatically and/or continuously.

## Revendications

1. Insert interchangeable (1) pour un appareil médical ophtalmologique (99) destiné à l'aspiration de liquide - lequel liquide doit en particulier être évacué dans le cadre d'un rinçage par aspiration lors d'une intervention chirurgicale ophtalmologique - comprenant :
o une zone de liquide (10) destinée à recevoir le liquide à aspirer et
o une zone de dépression (20) qui peut être soumise à une dépression,
dans lequel la zone de liquide (10) et la zone de dépression (20) sont séparées hermétiquement l'une de l'autre par une membrane (13) au moins partiellement flexible,
et lequel insert interchangeable (1) est conçu pour aspirer, au moyen de la dépression, le liquide dans la zone de liquide (10) dont le volume peut être modifié par déformation au moins partielle de la membrane.

2. Insert interchangeable (1) selon la revendication 1, **caractérisé en ce que** la membrane flexible (13) est conçue de telle sorte qu'il se produit, au moyen de la membrane flexible (13), une transmission de la dépression pneumatique dans la zone de dépression (20) à une dépression hydraulique dans la zone de liquide (10), et dans lequel l'insert interchangeable (1) est formé avec une demi-coque (11) avec laquelle la membrane (13) forme une cavité de volume variable qui constitue au moins une partie de la zone de liquide et présente un volume d'au moins approximativement zéro à plusieurs dizaines ou centaines de millilitres.

3. Insert interchangeable (1) selon la revendication 1 ou 2, **caractérisé en ce que** l'insert interchangeable (1) est formé avec une demi-coque (11) - en particulier constituée d'un thermoplastique tel qu'un polypropylène ou un polyéthylène - dans laquelle la membrane (13) - en particulier constituée d'un polymère tel qu'un polyamide et/ou un polypropylène - est disposée sous la forme d'une feuille flexible de telle sorte qu'une chambre d'aspiration (10) est formée entre la demi-coque (11) et un côté intérieur de la membrane (13), et que la zone de dépression (20) est formée sur un côté extérieur de la membrane (13), en particulier dans lequel un épaulement de la demi-coque (11), lorsque l'insert interchangeable (1) est inséré, forme avec une contrepartie (21) dans l'appareil médical (99) une chambre de dépression étanche périphériquement avec une aspiration d'air pour fournir la dépression, en particulier dans lequel une force de serrage est appliquée entre la contrepartie (21) et l'insert interchangeable (1) lorsque l'insert interchangeable (1) est inséré.

4. Insert interchangeable (1) selon au moins l'une des revendications 1, 2 ou 3, **caractérisé en ce que** l'insert interchangeable (1) est conçu comme une cassette interchangeable (50) pour l'appareil médical ophtalmologique (99), dans lequel la zone de liquide (10) forme une chambre d'aspiration pour recevoir au moins temporairement le liquide aspiré et peut être reliée hydrostatiquement à un raccord d'aspiration (12) d'un instrument chirurgical (91), et la zone de liquide (10) est formée avec
▪ la membrane flexible (13), qui est de préférence formée avec une surface assez grande pour que le rapport de surface de la membrane (13) sur la surface totale de la zone de liquide (10) soit supérieur à 16 %, en particulier supérieur à 25 % ou supérieur à 35 %, et
▪ une demi-coque fixe (11),
dans lequel la membrane (13), lorsque la cassette (50) est insérée dans l'appareil médical (99), peut être soumise à une dépression depuis l'extérieur par l'intermédiaire de la zone de dépression (20), de telle sorte qu'une transmission de dépression par l'intermédiaire de la membrane (13) puisse être effectuée.

5. Insert interchangeable (1) selon au moins l'une des revendications 1, 2 ou 3, **caractérisé en ce que** l'insert interchangeable (1) est conçu comme un module supplémentaire optionnel qui peut être monté sur une cassette de base ophtalmologique (51) qui présente déjà au moins une pompe péristaltique (60), en particulier dans lequel le module supplémentaire et la cassette de base (51) peuvent être encliquetés l'un avec l'autre et, lors du montage, une liaison hydraulique de la zone de liquide (10) de l'insert interchangeable (1) avec un système d'aspiration de la cassette de base (51) peut être établie.

6. Insert interchangeable (1) selon au moins l'une des revendications 1 à 5, **caractérisé en ce que** la dépression est produite par un moyen d'aspiration d'air (28) dans l'appareil médical (99), en particulier par une pompe à vide partiel actionnée électriquement ou une buse de Venturi, et dans lequel une valeur souhaitée de la dépression produite par le moyen d'aspiration d'air (28) peut être prédéfinie de manière variable dans le temps.

7. Insert interchangeable (1) selon au moins l'une des revendications 1 à 6, **caractérisé en ce que** la zone de liquide (10) présente un moyen de détection de pression (40) ou est en liaison hydrostatique avec un tel moyen, avec lequel une dépression dans la zone de liquide (10) peut être détectée.

8. Insert interchangeable (1) selon au moins l'une des revendications 1 à 7, **caractérisé en ce qu'**il est prévu dans l'insert interchangeable (1), dans un trajet de liquide entre la zone de liquide (10) et un raccord (12) pour une conduite d'aspiration (12), une vanne (65) avec laquelle une section transversale du trajet de liquide peut être modifiée, en particulier ouverte ou fermée.

9. Insert interchangeable (1) selon au moins l'une des revendications 1 à 8, **caractérisé en ce que** l'insert interchangeable (1) présente un dispositif de pompage (60) - en particulier une pompe péristaltique - avec lequel du liquide peut être évacué de la zone de liquide (10) dans un récipient à déchets interchangeable (33), de sorte que le dispositif de pompage (60) permet d'effectuer une vidange de la zone de liquide (10), le raccord d'aspiration (12) pouvant de préférence être séparé à cette fin de la zone de liquide (10) au moyen d'une vanne (65b) prévue à cet effet.

10. Insert interchangeable (1) selon au moins l'une des revendications 1 à 9, **caractérisé en ce que** des trajets de liquide sont formés dans l'insert interchangeable (1) de telle sorte que - en particulier au moyen d'au moins une vanne (65, 65a) prévue à cet effet - une commutation entre une aspiration par moins deux des moyens suivants :
▪ la dépression transmise à la zone de liquide (10),
▪ un dispositif de pompage alternatif (60), en particulier une pompe péristaltique, ou
▪ une combinaison de ces deux moyens
peut être effectuée,
en particulier dans lequel le dispositif de pompage alternatif (60) utilisé est le même dispositif de pompage (60) que celui de la revendication 7 avec lequel le liquide peut également être évacué dans le récipient à déchets (33).

11. Insert interchangeable (1) selon au moins l'une des revendications 1 à 10, **caractérisé en ce que** l'insert interchangeable (1) présente un capteur de niveau de remplissage de la zone de liquide (10) - en particulier conçu comme un capteur de pression (40) et/ou comme un capteur (31) pour déterminer une position de la membrane (13) - et/ou que l'insert interchangeable (1) présente un capteur de liquide ou d'humidité (32) dans la zone de dépression (20) - en particulier conçu pour détecter des défauts de la membrane (13) .

12. Appareil (50) pour l'opération des yeux avec un dispositif de réception pour un insert interchangeable (1) selon au moins l'une des revendications 1 à 11, l'appareil (99) présentant un moyen d'aspiration d'air (28) pour pomper de l'air hors de la zone de dépression (20) de l'insert interchangeable (1), qui est séparée hermétiquement de la zone de liquide (10) de l'insert interchangeable (1), en particulier dans lequel la moyen d'aspiration d'air (28) est réalisé avec une pompe à vide partiel ou une buse de Venturi, en particulier dans lequel le dispositif de réception pour l'insert interchangeable (1) est conçu de telle sorte qu'une liaison hermétique amovible d'une zone périphérique de l'insert interchangeable (1) avec une contrepartie (21) de l'appareil (99) soit réalisée pour former la zone de dépression fermée (20) .

13. Procédé d'aspiration d'un liquide, mis en œuvre dans un appareil ophtalmologique (99), en particulier une aspiration selon le principe de Venturi au moyen d'une aspiration par dépression pneumatique, comprenant :
▪ une mise à disposition d'une zone de liquide hermétiquement fermée (10), qui est au moins partiellement formée par une membrane flexible (13), dans un insert interchangeable (1) pour l'appareil ophtalmologique (99), et qui peut être mise en liaison hydraulique avec un raccord d'aspiration (12),
▪ une aspiration d'air sur un côté opposé à la zone de liquide (10) sur un côté extérieur de la membrane (13), lequel côté extérieur fait partie d'une zone de dépression (20),
▪ dans lequel la membrane (13) sépare hermétiquement la zone de liquide (10) de la zone de dépression (20) ainsi que de l'air ambiant,
▪ en particulier dans lequel une transmission d'une dépression pneumatique dans la zone de dépression (20) à une dépression hydraulique dans la zone de liquide (10) est effectuée au moyen de la membrane (13).

14. Procédé selon la revendication 13, **caractérisé par** une mesure d'une pression dans la zone de liquide (10) pour détecter des défauts et/ou un niveau de remplissage dans la zone de liquide (10) et/ou par une détection d'humidité ou de liquide dans la zone de dépression (20) pour détecter des défauts de la membrane (13).

15. Procédé selon la revendication 13 ou 14, **caractérisé en ce qu'**une commutation entre une aspiration :
▪ au moyen de la dépression pneumatique qui agit sur la zone de liquide (10) par l'intermédiaire de la membrane (13) et/ou
▪ au moyen d'un transport volumétrique hydraulique (60), en particulier avec une pompe péristaltique
peut être effectuée.

16. Procédé selon l'une des revendications 13 à 15, **caractérisé en ce qu'**une évacuation de liquide de la zone de liquide (10) dans un récipient à déchets (33) peut être effectuée, en particulier par pompage péristaltique, en particulier dans lequel la vidange de la zone de liquide (10) a lieu pendant des pauses d'aspiration, de préférence automatiquement et/ou de façon continue.
